# FASCICULE DE BREVET EUROPEEN

(11) **EP 1 279 398 B1**
(45) Date de publication et mention de la délivrance du brevet: **27.12.2006**
(21) Numéro de dépôt: 02291581.3
(22) Date de dépôt: 25.06.2002
(51) Int. Cl.: A61K 8/90

(54) **Composition à usage topique contenant un polymère diblocs**
Zusammensetzung zur lokalen Anwendung, die ein Diblock-Polymer enthält
Composition for topical use containing a diblock-polymer

(30) Priorité: 18.07.2001 FR 0109615
(43) Date de publication de la demande: 29.01.2003
(73) Titulaire: L'ORÉAL, 75008 Paris (FR)
(72) Inventeur: l'Alloret, Florence, 75013 Paris (FR)
(74) Mandataire: Rasson, Catherine

(56) Documents cités:
- EP-A- 1 052 267
- EP-A- 1 069 142
- WO-A-01/16187
- US-A- 5 756 080

## Description

La présente demande concerne une composition cosmétique et/ou dermatologique comprenant au moins une phase aqueuse comportant au moins un polymère diblocs, et à ses utilisations dans le domaine cosmétique ou dermatologique, notamment pour le soin, le nettoyage, la protection et/ou le maquillage des matières kératiniques (peau, muqueuses, fibres kératiniques telles que cheveux et cils).

Le plus souvent, les compositions cosmétiques, notamment celles destinées au soin ou au nettoyage de la peau humaine ou des cheveux, comprennent une phase aqueuse qui est gélifiée, c'est-à-dire épaissie, à l'aide d'un ou de plusieurs agent(s) épaississant(s) ou gélifiants. Il peut s'agir par exemple de lotions qui sont des solutions aqueuses ne contenant pas de phase huileuse, ou d'émulsions qui peuvent être des émulsions directes huile-dans-eau (H/E) comportant une phase grasse ou phase huileuse dispersée dans une phase continue aqueuse, ou des émulsions inverses eau-dans-huile (E/H) comportant une phase aqueuse dispersée dans une phase continue huileuse. On entend ici par « émulsions » aussi bien les dispersions obtenues en absence d'agents tensioactifs émulsionnants que les émulsions obtenues en présence d'agents tensioactifs émulsionnants.

Les émulsions huile-dans-eau sont les plus demandées dans le domaine de la cosmétique du fait qu'elles apportent à l'application sur la peau, un toucher plus doux, moins gras, plus frais et plus léger que les systèmes d'émulsions eau-dans-huile, grâce à la présence d'eau dans la phase externe continue.

La nature des composés utilisés pour gélifier la phase aqueuse et leur teneur dans la composition sont choisies en fonction du type de texture désiré, qui peut varier des lotions fluides aux émulsions plus ou moins épaisses pouvant constituer des laits ou des crèmes. Les principaux agents épaississants ou gélifiants utilisés dans le domaine cosmétique sont choisis parmi les composés suivants :
- les polymères naturels tels que les gommes de xanthane et de guar ou encore les dérivés cellulosiques, les amidons et les alginates. Ces composés n'apportent pas des propriétés cosmétiques suffisamment bonnes pour être utilisés seuls comme agent épaississant ou gélifiant. Leur origine naturelle peut d'autre part induire des problèmes de reproductibilité entre les lots de matière première, se traduisant par une variabilité du pouvoir gélifiant.
- les gélifiants polymériques réticulés tels que les Carbopols commercialisés par la société Goodrich ou les polymères d'acide 2-acrylamido 2-méthylpropane sulfonique réticulés et au moins partiellement neutralisés, comme par exemple le produit commercialisé sous la dénomination Hostacerin AMPS par la société Clariant. Toutefois, ces agents gélifiants réticulés nécessitent de suivre un protocole spécifique de dispersion dans l'eau ou dans la phase huileuse, afin d'obtenir des niveaux de viscosité reproductibles. Différents agents gélifiants ont été proposés afin de limiter ces problèmes de dispersions, comme par exemple les carbopols ETD qui sont des carbopols particuliers dits « faciles à disperser » (ETD = « Easy To Disperse ») ou encore les gélifiants réticulés dispersés dans une huile ou un mélange d'huiles, tels que le polyacrylamide commercialisé par la société Seppic sous la dénomination Sépigel 305. Toutefois, la dispersion dans l'eau des Carbopols ETD nécessite de suivre un protocole spécifique de gonflement du polymère, tandis que les gélifiants fournis en dispersion dans une huile introduisent obligatoirement dans la composition une phase huileuse et des tensioactifs.

Par ailleurs, les gélifiants cités ci-dessus ne possèdent pas de propriétés amphiphiles susceptibles de stabiliser les globules de la phase dispersée dans la phase continue d'une émulsion. On doit alors soit introduire des tensioactifs émulsionnants pour obtenir des émulsions stables soit n'introduire que de faibles teneurs en huile (en général inférieure à 10%) et concevoir des textures très gélifiées. Or, on recherche constamment à réduire la teneur en tensioactif émulsionnant dans les émulsions afin d'améliorer leur innocuité vis à vis de la peau, des yeux et du cuir chevelu. Par ailleurs, on cherche à pouvoir avoir la plus grande liberté possible dans la formulation, c'est-à-dire pouvoir obtenir une composition qui soit stable, quelle que soit la quantité d'huile introduite et quelle que soit la viscosité finale de la composition (gélification plus ou moins grande).

Il existe des gélifiants amphiphiles réticulés tels que les produits commercialisés par la société Goodrich sous la dénomination Pemulen, qui permettent d'incorporer des quantités d'huile plus importantes, mais ils sont utilisés en général en présence d'autres gélifiants hydrophiles car leurs propriétés gélifiantes et cosmétiques ne sont pas importantes et/ou bonnes.

Il subsiste donc le besoin de disposer d'un composé permettant de gélifier les phases aqueuses afin d'obtenir une large gamme de textures, et présentant de bonnes propriétés cosmétiques, qui soit facile à disperser dans l'eau et dont le pouvoir épaississant/gélifiant soit reproductible, et qui, par ailleurs, de manière préférée, présente à la fois des propriétés gélifiantes et émulsionnantes.

La demanderesse a découvert de façon inattendue une nouvelle famille de polymères blocs permettant d'atteindre le but de l'invention. Ces polymères blocs sont des polymères hydrosolubles ou hydrodispersibles, de structure diblocs A-B, où A est un bloc polymérique hydrosoluble ionique et B un bloc polymérique hydrophobe, dans lesquels la proportion en bloc polymérique ionique A est égale ou supérieure à 60 % en poids et de préférence supérieure à 70 % en poids par rapport au poids total du polymère. Ces polymères présentent des propriétés de gélification d'une phase aqueuse, à des concentrations en poids inférieures à 15 %. Ces polymères permettent d'obtenir une large gamme de textures ayant de bonnes propriétés cosmétiques. La solubilisation ou dispersion de ces polymères dans l'eau est facile et les propriétés de gélification obtenues sont reproductibles en fonction des différents lots.

Ces polymères permettent également de préparer des compositions cosmétiques ou dermatologiques comportant une phase aqueuse, dont le pH peut varier dans une large gamme, tout en ayant une viscosité qui reste stable dans le temps à température ambiante ou à des températures plus élevées. Ils permettent en outre de réaliser des produits homogènes, non-coulants, non-filants, doux et glissants à l'application et stables à la conservation.

La présente invention concerne une composition cosmétique et/ou dermatologique, caractérisée en ce qu'elle comprend au moins une phase aqueuse comportant au moins un polymère hydrosoluble ou hydrodispersible, de structure diblocs A-B où A est un bloc polymérique hydrosoluble ionique et B un bloc polymérique hydrophobe, la quantité de bloc polymérique A étant égale ou supérieure à 60 % du poids total du polymère diblocs.

La proportion d'au moins 60 % de bloc polymèrique A permet d'obtenir de bonnes propriétés de gélification du polymère.

En outre, selon un mode préféré de réalisation de l'invention, les polymères diblocs utilisés selon l'invention comme gélifiants ont un bloc polymérique hydrosoluble ionique A qui est totalement hydrosoluble, c'est-à-dire qui est complètement exempt de monomère hydrophobe, et un bloc polymérique hydrophobe B qui est totalement hydrophobe, c'est-à-dire qui est complètement exempt de monomère hydrophile.

Les polymères diblocs préférés, comportant un bloc polymérique ionique A totalement hydrosoluble et un bloc polymérique B totalement hydrophobe présentent l'avantage d'être faciles à synthétiser et de donner une aussi bonne gélification que les autres pour des concentrations moindres.

Ainsi, la composition selon l'invention comprend de manière préférée au moins une phase aqueuse comportant au moins un polymère hydrosoluble ou hydrodispersible, de structure diblocs A-B où A est un bloc polymérique ionique totalement hydrosoluble et B un bloc polymérique totalement hydrophobe, la quantité de bloc polymérique A étant égale ou supérieure à 60 % du poids total du polymère diblocs.

Les polymères diblocs utilisés selon l'invention et notamment les polymères préférés comportant un bloc polymérique ionique A totalement hydrosoluble et un bloc polymérique B totalement hydrophobe, permettent d'obtenir la gélification satisfaisante des phases aqueuses des compositions pour application topique, notamment cosmétiques ou dermatologiques.

Aussi, l'invention a encore pour objet l'utilisation d'au moins un polymère hydrosoluble ou hydrodispersible, de structure diblocs A-B où A est un bloc polymérique hydrosoluble ionique et B un bloc polymérique hydrophobe, la quantité de bloc polymérique A étant égale ou supérieure à 60 % du poids total du polymère diblocs, pour la gélification d'une composition cosmétique ou dermatologique comprenant au moins une phase aqueuse.

De manière préférée, l'invention a aussi pour objet l'utilisation d'au moins un polymère hydrosoluble ou hydrodispersible, de structure diblocs A-B où A est un bloc polymérique totalement hydrosoluble ionique et B un bloc polymérique totalement hydrophobe, la quantité de bloc polymérique A étant égale ou supérieure à 60 % du poids total du polymère diblocs, pour la gélification d'une composition cosmétique ou dermatologique comprenant au moins une phase aqueuse.

Comme décrit ci-dessus, les polymères diblocs utilisés selon l'invention permettent d'obtenir une bonne gélification des phases aqueuses. Par ailleurs, ce pouvoir gélifiant présente l'avantage d'être peu modifié en présence de tensioactifs, que ceux-ci soient non ioniques ou ioniques (anioniques ou cationiques) et que ce soient des tensioactifs émulsionnants ou des tensioactifs détergents (ou tensioactifs moussants). Quand la quantité de tensioactif est élevée (supérieur à 1 %), le pouvoir gélifiant subsiste même s'il peut être diminué.

Par ailleurs, la demanderesse a trouvé de manière inattendue que les polymères de l'invention de structure diblocs A-B où A est un bloc polymérique hydrosoluble ionique et B un bloc polymérique hydrophobe, la quantité de bloc polymérique A étant égale ou supérieure à 60 % du poids total du polymère diblocs, avaient des propriétés émulsionnantes et pouvaient également être utilisés pour la préparation d'émulsion sans tensioactif émulsionnant ou contenant de faibles teneurs en tensioactif émulsionnant (de 0 à environ 1% du poids total de la composition, et de préférence de moins de 0,5 % en poids).

Aussi, l'invention a encore pour objet une composition cosmétique et/ou dermatologique sous forme d'émulsion huile-dans-eau, caractérisée en ce qu'elle comprend de 0 à environ 1 % de tensioactif émulsionnant et qu'elle est conforme à la composition définie ci-dessus, c'est-à-dire que la phase aqueuse comprend un polymère hydrosoluble ou hydrodispersible, de structure diblocs A-B où A est un bloc polymérique hydrosoluble ionique et B un bloc polymérique hydrophobe, la quantité de bloc polymérique A étant égale ou supérieure à 60 % du poids total du polymère diblocs. L'émulsion obtenue est considérée comme une émulsion exempte de tensioactif émulsionnant.

Par ailleurs, la demanderesse a trouvé également de manière inattendue qu'il se produisait une synergie de gélification quand on utilisait un polymère hydrosoluble ou hydrodispersible, de structure diblocs A-B où A est un bloc polymérique hydrosoluble ionique et B un bloc polymérique hydrophobe, la quantité de bloc polymérique A étant égale ou supérieure à 60 % du poids total du polymère diblocs, avec un polymère hydrosoluble ou hydrodispersible diblocs neutre A'-B dans lequel A' est un bloc polymérique hydrosoluble neutre et B un bloc polymérique hydrophobe.

Aussi, l'invention a encore pour objet une composition cosmétique et/ou dermatologique, caractérisée en ce qu'elle comprend au moins une phase aqueuse comportant au moins un polymère hydrosoluble ou hydrodispersible, de structure diblocs A-B où A est un bloc polymérique hydrosoluble ionique et B un bloc polymérique hydrophobe, la quantité de bloc polymérique A étant égale ou supérieure à 60 % du poids total du polymère diblocs, et au moins un polymère hydrosoluble ou hydrodispersible diblocs neutre A'-B dans lequel A' est un bloc polymérique hydrosoluble neutre et B un bloc polymérique hydrophobe.

Par « hydrosoluble ou hydrodispersible », on entend dans la présente demande, des polymères qui, introduits dans une phase aqueuse à 25°C, à une concentration en poids égale à 1%, permettent l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 70%, de préférence d'au moins 80%.

Par « bloc polymérique », on entend dans la présente demande un polymère (homopolymère ou copolymère) dont la masse molaire est supérieure à 400 g/mole, et de préférence supérieure à 800 g/mole.

Par « bloc hydrophobe », on entend dans la présente demande, un polymère (homopolymère ou copolymère) qui, introduit dans un solvant hydrocarboné à 25°C, à une concentration en poids égale à 1%, permet l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 70%, de préférence d'au moins 80%. Le solvant hydrocarboné utilisé ici a une constante diélectrique mesurée à 25°C, inférieure à 50 ; ce solvant peut être notamment choisi parmi les alcanes tels que le cyclohexane (constante diélectrique : 2,02) ; les solvants aromatiques tels que l'éthylbenzène (constante diélectrique : 2,4) ; les cétones telles que la cyclohexanone (constante diélectrique : 18,3) ; les éthers tels que le diéthyléther (constante diélectrique = 4,4) ; les alcools tels que le cyclohexanol (constante diélectrique : 15,0) ; les solvants chlorés hydrocarbonés tels que le dichlorométhane (constante diélectrique: 9,08) ; les amides telles que la diméthylformamide ; et les esters tels que l'acétate d'éthyle (constante diélectrique : 6,02).

Les compositions de l'invention étant destinées à une application topique contiennent un milieu physiologiquement acceptable, c'est-à-dire un milieu compatible avec toutes les matières kératiniques telles que la peau, les ongles, les muqueuses et les cheveux ou toute autre zone cutanée du corps.

Dans les polymères hydrosolubles ou hydrodispersibles de structure diblocs A-B, utilisés dans la composition de l'invention, le bloc hydrosoluble ionique A est obtenu à partir d'un ou plusieurs monomères hydrosolubles (la), ou leurs sels, comme par exemple :
- l'acide (méth)acrylique,
- l'acide styrène sulfonique,
- l'acide vinylsulfonique et l'acide (méth)allylsulfonique,
- l'acide vinyl phosphonique,
- l'acide maléique,
- l'acide itaconique,
- l'acide crotonique,
- le chlorure de diméthyldiallyl ammonium,
- le chlorure de méthylvinylimidazolium,
- les carboxybétaïnes ou sulfobétaïnes éthyléniques, obtenues par exemple par quaternisation de monomères à insaturation éthyléniques comportant une fonction amine, par des sels de sodium d'acide carboxylique à halogène mobile, comme le chloroacétate de sodium, ou par des sulfones cycliques comme le propane sulfone,
- les monomères vinyliques hydrosolubles de formule (I) suivante : 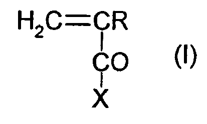
dans laquelle :
- R est choisi parmi H, -CH₃, -C₂H₅ ou -C₃H₇ ;
- X est choisi parmi :
   - les oxydes d'alkyle de type -OR₁ où R₁ est un radical hydrocarboné, linéaire ou ramifié, saturé ou insaturé, ayant de 1 à 6 atomes de carbone, substitué par au moins un groupement carboxylate (CO₂⁻) et/ou sulfonique (-SO₃⁻) et/ou sulfate (-SO₄⁻) et/ou phosphate (-PO₄H₂⁻) et/ou ammonium quaternaire (-N⁺R₂R₃R₄), les radicaux R₂, R₃ et R₄ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₂ + R₃ + R₄ ne dépasse pas 6. En outre, le radical R₁ peut être éventuellement substitué par un atome d'halogène (iode, brome, chlore, fluor) ; un groupement hydroxy (-OH) ; acide carboxylique (-COOH) ; éther (-O-); amine primaire (-NH₂) ; amine secondaire (-NHR₅) ; amine tertiaire (-NR₅R₆), les radicaux R₅ et R₆ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₅ + R₆ ne dépasse pas 6.
      Comme monomères vinyliques comportant des groupes ester (X=OR₁), on peut citer par exemple le méthacrylate de diméthylaminoéthyl quaternisé (MADAME).
   - les groupements -NH₂, -NHR₇ et -NR₇R₈ dans lesquels R₇ et R₈ sont, indépendamment l'un de l'autre, un radical hydrocarboné, linéaire ou ramifié, saturé ou insaturé ayant 1 à 6 atomes de carbone, sous réserve que le nombre total d'atomes de carbone de R₇ + R₈ ne dépasse pas 6, lesdits radicaux R₇ et/ou R₈ étant substitués par au moins un groupement carboxylate (-COO⁻) et/ou sulfonique (-SO₃⁻) et/ou sulfate (-SO₄⁻) et/ou phosphate (-PO₄H₂⁻) et/ou amine quaternaire (-N⁺R₉R₁₀R₁₁), les radicaux R₉, R₁₀ et R₁₁ étant, indépendamment l'un de l'autre, un radical hydrocarboné, linéaire ou ramifié, saturé ou insaturé ayant 1 à 6 atomes de carbone, sous réserve que la somme des atomes de carbone de R₇ + R₈ + R₉ + R₁₀ + R₁₁ ne dépasse pas 6. En outre, les radicaux R₇ et/ou R₈ peuvent éventuellement être substitués par un atome d'halogène (iode, brome, chlore, fluor) ; ou un groupement acide carboxylique (-COOH), hydroxy (-OH) ; éther (-O-); amine primaire (-NH₂) ; amine secondaire (-NHR₅) ; ou amine tertiaire (-NR₅R₆) où R₅ et R₆ ont les significations indiquées ci-dessus, sous réserve que la somme des atomes de carbone de R₇ + R₈ + R₅ + R₆ ne dépasse pas 6.

Comme monomères vinyliques comportant des groupes amide, on peut citer par exemple l'acide 2-acrylamido-2-méthylpropane sulfonique (AMPS), le chlorure de (méth)acrylamidopropyltriméthylammonium (APTAC et MAPTAC).
- les substituants R et X étant tels que le monomère de formule (I) soit hydrosoluble.
- et les mélanges de ces monomères (la).

A côté des monomères hydrosolubles (la) indiqués ci-dessus et toujours présents, le bloc hydrosoluble ionique A des polymères de l'invention peut éventuellement être obtenu à partir d'un ou plusieurs monomères choisis parmi les monomères hydrophobes (lb), les monomères hydrosolubles neutres (lc) et leurs mélanges. Les monomères hydrophobes éventuellement présents doivent être en une quantité suffisamment faible pour que le bloc A soit hydrosoluble.

On peut citer par exemple comme monomères hydrophobes (lb) utilisables dans le bloc hydrosoluble ionique A :
- le styrène et ses dérivés tel que le 4-butylstyrène, l'alpha-méthylstyrène et le vinyltoluène ;
- l'acétate de vinyle de formule CH₂=CH-OCOCH₃ ;
- les vinyléthers de formule CH₂=CHOR₁₂ dans laquelle R₁₂ est un radical hydrocarboné, linéaire ou ramifié, saturé ou insaturé, ayant de 1 à 6 atomes de carbone ;
- l'acrylonitrile ;
- la caprolactone ;
- le chlorure de vinyle et le chlorure de vinylidène ;
- les monomères siliconés insaturés tels que le méthacryloxypropyl-tris(triméthylsiloxy)silane et les méthacrylamides siliconés,
- les monomères vinyliques hydrophobes de formule (II) suivante : 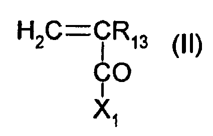 dans laquelle :
   - R₁₃ est choisi parmi H, -CH₃, -C₂H₅ ou -C₃H₇ ;
   - X₁ est choisi parmi :
      - les oxydes d'alkyle de type -OR₁₄ où R₁₄ est un radical hydrocarboné, linéaire ou ramifié, saturé ou insaturé, ayant de 1 à 6 atomes de carbone. On peut citer comme monomères vinyliques comportant des groupes esters, le méthacrylate de méthyle, le méthacrylate d'éthyle, le (meth)acrylate de n-butyle, le (meth)acrylate de tertio-butyle, l'acrylate de cyclohexyle, l'acrylate d'isobornyle et l'acrylate d'éthyle 2-hexyle ;
      - les groupements -NN₂, -NHR₁₅ et -NR₁₅R₁₆ dans lesquels R₁₅ et R₁₆ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₅ + R₁₆ ne dépasse pas 6 ;
      - les substituants R₁₃ et X₁ étant tels que le monomère de formule (II) soit hydrophobe.
      - et les mélanges de ces monomères.

Comme monomères neutres hydrosolubles (lc), on peut citer par exemple :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyle cyclique ayant de 4 à 9 atomes de carbone, tels que la N-vinylpyrrolidone, la N-butyrolactame et la N-vinylcaprolactame,
- l'alcool vinylique de formule CH₂=CHOH,
- les monomères vinyliques hydrosolubles de formule (III) suivante : 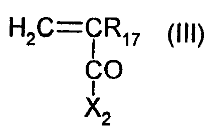 dans laquelle :
   - R₁₇ est choisi parmi H, -CH₃, -C₂H₅ ou -C₃H₇
   - X₂ est choisi parmi :
      - les oxydes d'alkyle de type -OR₁₈ où R₁₈ est un radical hydrocarboné, linéaire ou ramifié, saturé ou insaturé, ayant de 1 à 6 atomes de carbone, éventuellement substitué par un atome d'halogène (iode, brome, chlore, fluor) ; un groupement acide carboxylique (-COOH), hydroxy (-OH) ; éther (-O-) ; amine primaire (-NH₂) ; amine secondaire (-NHR₁₉), tertiaire (-NR₁₉R₂₀), les radicaux R₁₉ et R₂₀ étant, indépendamment l'un de l'autre, un radical hydrocarboné, linéaire ou ramifié, saturé ou insaturé ayant 1 à 6 atomes de carbone, sous réserve que la somme des atomes de carbone de R₁₈ + R₁₉ + R₂₀ ne dépasse pas 6. On peut citer par exemple comme monomères de formule (III) où X₂ est un radical -OR₁₈, le (méth)acrylate de glycidyle, le méthacrylate d'hydroxyéthyle, et les (méth)acrylates d'éthylène glycol, de diéthylèneglycol ou de polyalkylèneglycol.
      - les groupements -NH₂, -NHR₂₁ et -NR₂₁R₂₂ dans lesquels R₂₁ et R₂₂ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₂₁ + R₂₂ ne dépasse pas 6, lesdits radicaux R₂₁ et R₂₂ étant éventuellement substitués par un atome d'halogène (iode, brome, chlore, fluor) ; un groupement hydroxy (-OH) ; carboxylique (CO₂H) ; éther (-O-); amine primaire (-NH₂) ; amine secondaire (-NHR₂₃) ; ou amine tertiaire (-NR₂₃R₂₄), les radicaux R₂₃ et R₂₄ étant, indépendamment l'un de l'autre, un radical hydrocarboné, linéaire ou ramifié, saturé ou insaturé ayant 1 à 6 atomes de carbone, sous réserve que la somme des atomes de carbone de R₂₁ + R₂₂ + R₂₃ + R₂₄ ne dépasse pas 6. On peut citer par exemple comme monomères de ce type, le diméthylamino-éthylméthacrylamide et la N,N-diméthylacrylamide ;
      - les substituants R₁₇ et X₂ étant tels que le monomère de formule (III) soit hydrosoluble ;
      - les mélanges de ces monomères.

Outre les monomères hydrosolubles indiqués ci-dessus, le bloc hydrosoluble ionique A peut également être un polymère hydrosoluble ionique tel que par exemple la polyéthylène imine.

Le bloc hydrosoluble ionique A est totalement ou partiellement neutralisé. On entend par « partiellement neutralisé » une neutralisation d'au moins 20 % en mole. Le bloc hydrosoluble ionique A peut être neutralisé par une base minérale ou organique. Cette base peut être choisie, par exemple, parmi les sels de sodium, ammonium, lithium, calcium, magnésium, ammonium substitué par 1 à 4 groupes alkyle portant de 1 à 15 atomes de carbone, ou encore parmi les bases organiques comme la mono-, la di-, et la triéthanolamine, l'aminoéthyl-propanediol, la N-méthyl-glucamine, et les acides aminés basiques, tels que l'arginine et la lysine, et leurs mélanges.

Le bloc hydrophobe B est obtenu à partir d'un ou plusieurs monomères hydrophobes (Id), tels que par exemple :
- le styrène et ses dérivés tel que le 4-butylstyrène, l'alpha-méthylsyrène et le vinyltoluène,
- l'acétate de vinyle de formule CH₂=CH-OCOCH₃,
- les vinyléthers de formule CH₂=CHOR₂₅ dans laquelle R₂₅ est un radical hydrocarboné, linéaire ou ramifié, saturé ou insaturé, ayant de 1 à 22 atomes de carbone,
- l'acrylonitrile,
- le chlorure de vinyle et le chlorure de vinylidène,
- la caprolactone,
- les alcènes tels que l'éthylène, le propylène, le butylène et le butadiène,
- les monomères siliconés insaturés tels que le méthacryloxypropyl-tris(triméthylsiloxy)silane et les méthacrylamides siliconés, ainsi que les dérivés siliconés conduisant après polymérisation à des polymères siliconés tel que le polydiméthylsiloxane,
- les monomères vinyliques hydrophobes de formule (IV) suivante : 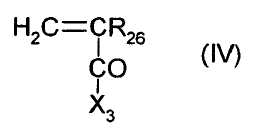 dans laquelle :
   - R₂₆ est choisi parmi H, -CH₃, -C₂H₅ ou -C₃H₇ ;
   - X₃ est choisi parmi :
      - les oxydes d'alkyle de type -OR₂₇ où R₂₇ est un radical hydrocarboné, linéaire ou ramifié, saturé ou insaturé, ayant de 1 à 22 atomes de carbone, éventuellement substitué par un atome d'halogène (iode, brome, chlore, fluor) ; un groupement carboxylate (-CO₂⁻), sulfonique (-SO₃⁻), sulfate (-SO₄⁻), phosphate (-PO₄H₂⁻), hydroxy (-OH), acide carboxylique (-COOH), éther (-O-), amine primaire (-NH₂), amine secondaire (-NHR₂₈), amine tertiaire (-NR₂₈R₂₉) ou ammonium quaternaire (-N⁺R₂₈R₂₉R₃₀), les radicaux R₂₈, R₂₉ et R₃₀ étant, indépendamment l'un de l'autre, un radical hydrocarboné, linéaire ou ramifié, saturé ou insaturé ayant 1 à 22 atomes de carbone, sous réserve que la somme des atomes de carbone de R₂₇ + R₂₈ + R₂₉+ R₃₀ ne dépasse pas 22 ; ou bien R₂₇ est un radical perfluoroalkyle, comportant de préférence de 1 à 18 atomes de carbone ;

Comme monomères vinyliques hydrophobes comportant des groupements oxydes d'alkyle de type -OR₂₇, on peut citer par exemple, le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle. Comme monomère de formule (IV) avec un radical perfluoroalkyle constituant le groupe R₂₇, on peut citer par exemple l'acrylate d'éthyle perfluorooctyle et le (méth)acrylate de trifluorométhyle ;
- les groupements -NH₂, -NHR₃₁ et -NR₃₁R₃₂ dans lesquels les radicaux R₃₁ et R₃₂ sont, indépendamment l'un de l'autre, un radical hydrocarboné, linéaire ou ramifié, saturé ou insaturé ayant 1 à 22 atomes de carbone, sous réserve que le nombre total d'atomes de carbone de R₃₁ + R₃₂ ne dépasse pas 22, lesdits radicaux R₃₁ et R₃₂ étant éventuellement substitués par un atome d'halogène (iode, brome, chlore, fluor) ; un groupement hydroxy (-OH), éther (-O-), carboxylate (-CO₂⁻), sulfonique (-SO₃⁻), sulfate (-SO₄⁻), phosphate (-PO₄H₂⁻), acide carboxylique (-COOH), amine primaire (-NH₂), amine secondaire (-NHR₂₈), amine tertiaire (-NR₂₈R₂₉), ou ammonium quaternaire (-N⁺R₂₈R₂₉R₃₀) où R₂₈, R₂₉ et R₃₀ ont les mêmes significations que celles indiquées ci-dessus, sous réserve que la somme des atomes de carbone de R₃₁ + R₃₂+ R₂₈+ R₂₉ R₃₀ ne dépasse pas 22. R₃₁ et R₃₂ indépendamment l'un de l'autre peuvent également être un radical perfluoroalkyle, comportant de préférence de 1 à 18 atomes de carbone.
- les substituants R₂₆ et X₃ étant tels que le monomère de formule (IV) soit hydrophobe ;
- et les mélanges de ces monomères.

A côté des monomères hydrophobes (Id) indiqués ci-dessus toujours présents, le bloc hydrophobe B des polymères de l'invention peut éventuellement être obtenu à partir d'un ou plusieurs monomères hydrosolubles ioniques ou neutres (le), les dits monomères hydrosolubles étant présents en une quantité suffisamment faible pour que le bloc B soit hydrophobe.

Comme monomères hydrosolubles (le), on peut citer par exemple les composés suivants ou leurs sels :
- l'acide (méth)acrylique ;
- l'acide styrène sulfonique ;
- l'acide vinylsulfonique et l'acide (méth)allylsulfonique ;
- l'acide vinyl phosphonique ;
- l'anhydride maléique ;
- l'acide maléfique ;
- l'acide itaconique ;
- l'acide crotonique ;
- le chlorure de diméthyldiallyl ammonium ;
- le chlorure de méthylvinylimidazolium ;
- le (méth)acrylamide ;
- la N-vinylacétamide et la N-méthyl N-vinylacétamide ;
- la N-vinylformamide et la N-méthyl N-vinylformamide ;
- les N-vinyllactames comportant un groupe alkyle cyclique ayant de 4 à 9 atomes de carbone, tels que la N-vinylpyrrolidone, la N-butyrolactame et la N-vinylcaprolactame,
- l'alcool vinylique de formule CH₂=CHOH ;
- la 2-vinylpyridine et la 4-vinylpyridine ;
- les monomères vinyliques hydrosolubles de formule (V) suivante : 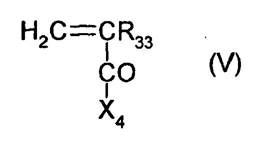 dans laquelle :
   - R₃₃ est choisi parmi H, -CH₃, -C₂H₅ ou -C₃H₇;
   - X₄ est choisi parmi :
      - les oxydes d'alkyle de type -OR₃₄ où R₃₄ est un radical hydrocarboné, linéaire ou ramifié, saturé ou insaturé, ayant de 1 à 6 atomes de carbone, éventuellement substitué par un atome d'halogène (iode, brome, chlore, fluor); un groupement carboxylate (-COO⁻), acide carboxylique (-COOH), sulfonique (-SO₃⁻), sulfate (-SO₄⁻), phosphate (-PO₄H₂⁻), hydroxy (-OH), éther (-O-), amine primaire (-NH₂), amine secondaire (-NHR₃₅), amine tertiaire (-NR₃₅R₃₆) ou ammonium quaternaire (-N⁺R₃₅R₃₆R₃₇), les radicaux R₃₅, R₃₆ et R₃₇ étant, indépendamment l'un de l'autre, un radical hydrocarboné, linéaire ou ramifié, saturé ou insaturé ayant 1 à 6 atomes de carbone, sous réserve que la somme des atomes de carbone de R₃₄ + R₃₅ + R₃₆ + R₃₇ ne dépasse pas 6.
         Comme monomères vinyliques comportant des groupes ester (X₄=OR₃₄), on peut citer par exemple le méthacrylate de diméthylaminoéthyl quaternisé (MADAME), le (méth)acrylate de glycidyle, le méthacrylate d'hydroxyéthyle, et les (méth)acrylates d'éthylène glycol, de diéthylèneglycol ou de polyalkylèneglycol.
      - les groupements -NH₂, -NHR₃₈ et -NR₃₈R₃₉ dans lesquels les radicaux R₃₈ et R₃₉ sont, indépendamment l'un de l'autre, des radicaux hydrocarbonés, linéaires ou ramifiés, saturés ou insaturés ayant 1 à 6 atomes de carbone, sous réserve que le nombre total d'atomes de carbone de R₃₈ + R₃₉ ne dépasse pas 6, lesdits radicaux R₃₈ et/ou R₃₉ étant éventuellement substitués par un atome d'halogène (iode, brome, chlore, fluor); un groupement carboxylate (-COO⁻), sulfonique (-SO₃⁻); sulfate (-SO₄⁻) ; phosphate (-PO₄H₂⁻) ; hydroxy (-OH); acide carboxylique (-COOH), éther (-O-); amine primaire (-NH₂) ; amine secondaire (-NHR₃₅), amine tertiaire (-NR₃₅R₃₆) ou un ammonium quaternaire (-N+R₃₅R₃₆R₃₇) où R₃₅, R₃₆ et R₃₇ ont la même signification qu'indiquée ci-dessus, sous réserve que la somme des atomes de carbone de R₃₈ + R₃₉ + R₃₅ + R₃₆ + R₃₇ ne dépasse pas 6.
         Comme monomères vinyliques hydrosolubles de formule (V) comportant des groupes amide, on peut citer par exemple l'acide 2-acrylamido-2-méthylpropane sulfonique (AMPS), le chlorure de (méth)acrylamido-propyltriméthylammonium (APTAC et MAPTAC) et la N,N diméthylacrylamide.
      - les substituants R₃₃ et X₄ étant tels que le monomère de formule (V) soit hydrosoluble.
      - et les mélanges de ces monomères (le).

Les polymères diblocs utilisés dans la composition de l'invention ont une masse molaire allant de 1 000 g/mole à 500 000 g/mole, de préférence de 2 000 g/mole à 300 000 g/mole. Le bloc hydrosoluble ionique A a une masse molaire allant de 600 g/mole à 300 000 g/mole, de préférence de 1 200 g/mole à 180 000 g/mole. Le bloc hydrophobe B a une masse molaire allant de 400 g/mole à 200 000 g/mole, de préférence de 800 g/mole à 120 000 g/mole.

Pour avoir un polymère hydrosoluble, la proportion du bloc hydrophile ionique A dans le polymère de l'invention est égale ou supérieure à 60 % en poids, et elle est de préférence égale ou supérieure à 70 % en poids par rapport au poids total du polymère diblocs (blocs A + B).

Selon un mode préféré de réalisation de l'invention, si le polymère bloc utilisé dans la composition de l'invention contient un noyau aromatique, ce noyau n'est présent que dans un seul bloc (A ou B) mais jamais simultanément dans les blocs A et B.

Selon un mode particulier de réalisation de l'invention, le polymère diblocs est un polymère polystyrène (2000 g/mole) - Polyacrylate de sodium (11500 g/mole).

Les polymères blocs de l'invention peuvent être préparés par les procédés de synthèse classiquement utilisés pour obtenir des polymères blocs. On peut citer par exemple comme procédé de préparation, les polymérisations de type anionique ou cationique, et la polymérisation radicalaire contrôlée (voir "New Method of Polymer Synthesis", Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la voie par transfert d'atomes (Atom Transfert Radical Polymerization ou ATRP) (voir JACS, 117, page 5614 (1995), de Matyjasezwski et al.), la méthode des radicaux tels que les nitroxydes (Georges et al., Macromolecules, 1993, 26, 2987) ou encore la voie par transfert de chaîne réversible avec addition-fragmentation (Radical Addition-Fragmentation chain Transfert) telle que le procédé MADIX (Macromolecular Design via the Interchange of Xanthate) (Charmot D., Corpart P., Adam H., Zard S.Z., Biadatti T., Bouhadir G., Macromol. Symp., 2000, 150, 23). Les polymères diblocs utilisés dans la composition de l'invention peuvent être obtenus par ces procédés de synthèse. On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs A et B.

La quantité de polymère(s) diblocs dans la composition de l'invention peut varier selon le type de composition que l'on veut obtenir et le degré de gélification souhaitée. Elle peut aller par exemple de 0,01 à 20 % en poids de matière active, de préférence de 0,05 à 15 % en poids et mieux de 0,1 à 10 % en poids par rapport au poids total de la composition.

Comme indiqué plus haut, on observe une synergie quand les polymères diblocs A-B sont utilisés avec des polymères diblocs A'-B comprenant un bloc polymérique hydrosoluble neutre A et un bloc polymérique hydrophobe B. Ainsi, les polymères de l'invention peuvent être utilisés comme agents gélifiants, soit seuls (un ou plusieurs polymères diblocs A-B) soit en association avec un ou plusieurs polymères hydrosolubles ou hydrodispersibles neutres, de structure diblocs A'-B où A' est un bloc polymérique hydrosoluble neutre et B un bloc polymérique hydrophobe tel que défini ci-dessus pour le polymère diblocs. La quantité du polymère diblocs ionique A-B dans le mélange de polymères diblocs ionique A-B et de polymères diblocs neutres A'-B est supérieure à 10 % en poids et de préférence supérieure à 20 % en poids par rapport à la quantité totale de polymères diblocs, ce qui signifie que sur 100 % de mélange, il doit y avoir au moins 10 % de polymère(s) diblocs ionique A-B.

Dans les polymères hydrosolubles ou hydrodispersibles neutres A'-B, le bloc polymérique hydrophobe B a la même définition que celle donnée précédemment pour les polymères diblocs A-B.

Le bloc hydrosoluble neutre A' peut être un polymère (homopolymère ou copolymère) polyoxyalkyléné et notamment polyoxyéthyléné ou polyoxypropyléné, comme par exemple le poly-oxyde d'éthylène (POE), le poly-oxyde de propylène (POP), les copolymères d'oxyde d'éthylène (OE), d'oxyde de propylène (OP) et leurs mélanges.

Le bloc hydrosoluble neutre A' peut également être obtenu à partir d'un ou plusieurs monomères hydrosolubles (If) et leurs mélanges, comme par exemple :
- le (méth)acrylamide,
- la N-vinylacétamide et la N-méthyl N-vinylacétamide,
- la N-vinylformamide et la N-méthyl N-vinylformamide,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'alcool vinylique de formule CH₂=CHOH,
- l'anhydride maléique,
- la vinylamine,
- les monomères vinyliques hydrosolubles de formule (VI) suivante : 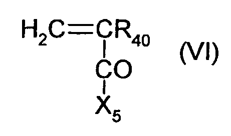 dans laquelle :
   - R₄₀ est choisi parmi H, -CH₃, -C₂H₅ ou -C₃H₇;
   - X₅ est choisi parmi :
      - les oxydes d'alkyle de type -OR₄₁ où R₄₁ est un radical hydrocarboné, linéaire ou ramifié, saturé ou insaturé, ayant de 1 à 6 atomes de carbone, éventuellement substitué par un atome d'halogène (iode, brome, chlore, fluor); un groupement hydroxy (-OH) ; acide carboxylique (-COOH), éther (-O-); amine primaire (-NH₂) ; amine secondaire (-NHR₄₂), ou amine tertiaire (-NR₄₂R₄₃), les radicaux R₄₂ et R₄₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄₁ + R₄₂ + R₄₃ ne dépasse pas 6.
         Comme monomères vinyliques hydrosolubles de formule (VI) comportant des groupes esters, on peut citer par exemple le (méth)acrylate de glycidyle, le méthacrylate d'hydroxyéthyle, et les (méth)acrylates d'éthylène glycol, de diéthylèneglycol ou de polyalkylèneglycol.
      - les groupements -NH₂, -NHR₄₄ et -NR₄₄R₄₅ dans lesquels R₄₄ et R₄₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄₄ + R₄₅ ne dépasse pas 6, lesdits radicaux R₄₄ et R₄₅ étant éventuellement substitués par un atome d'halogène (iode, brome, chlore, fluor) ; un groupement acide carboxylique (-COOH) ; hydroxy (-OH) ; éther (-O-); amine primaire (-NH₂) ; amine secondaire (-NHR₄₆) ; ou amine tertiaire (-NR₄₆R₄₇), les radicaux R₄₆ et R₄₇ étant, indépendamment l'un de l'autre, un radical hydrocarboné, linéaire ou ramifié, saturé ou insaturé ayant 1 à 6 atomes de carbone, sous réserve que la somme des atomes de carbone de R₄₄ + R₄₅ + R₄₆ et R₄₇ ne dépasse pas 6.
         Comme monomères vinyliques hydrosolubles de formule (VI) comportant des groupes amides, on peut citer par exemple la N,N diméthylacrylamide.
      - les groupes R₄₀ et X₅ étant tels que le monomère de formule (VI) soit hydrosoluble ;
      - et les mélanges de ces monomères (If).

A côté des monomères (If), le bloc hydrosoluble neutre A' peut éventuellement également être obtenu à partir des monomères hydrophobes (Ig), les dits monomères hydrophobes étant présents en une quantité suffisamment faible par rapport aux monomères (If) pour que le bloc A' soit soluble dans l'eau.

Comme monomères hydrophobes (Ig), on peut citer par exemple :
- le styrène et ses dérivés tel que la 4-butylstyrène, l'alpha-méthylstyrène et le vinyltoluène,
- l'acétate de vinyle de formule CH₂=CH-OCOCH₃ ;
- les vinyléthers de formule CH₂=CHOR₄₈ dans laquelle R₄₈ est un radical hydrocarboné, linéaire ou ramifié, saturé ou insaturé, ayant de 1 à 6 atomes de carbone ;
- l'acrylonitrile,
- la caprolactone,
- le chlorure de vinyle et le chlorure de vinylidène,
- les monomères siliconés insaturés, tels que le méthacryloxypropyl-tris(triméthylsiloxy)silane et les méthacrylamides siliconés,
- les monomères vinyliques hydrophobes de formule (VII) suivante : 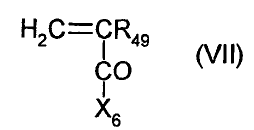 dans laquelle :
   - R₄₉ est choisi parmi H, -CH₃, -C₂H₅ ou -C₃H₇;
   - X₆ est choisi parmi :
      - les oxydes d'alkyle de type -OR₅₀ où R₅₀ est un radical hydrocarboné, linéaire ou ramifié, saturé ou insaturé, ayant de 1 à 6 atomes de carbone. Comme monomères vinyliques hydrophobes de formule (VII) comportant des groupes esters, on peut citer par exemple le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle ;
      - les groupements -NH₂, -NHR₅₁ et -NR₅₁R₅₂ dans lesquels R₅₁ et R₅₂ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₅₁ + R₅₂ ne dépasse pas 6 ;
      - les substituants R₄₉ et X₆ étant tels que le monomères de formule (VII) soit hydrophobe ;
      - et les mélanges de ces monomères (lg).

Les polymères diblocs neutres A'-B ont une masse molaire allant de 1 000 g/mole et 500 000 g/mole, de préférence de 2 000 g/mole à 300 000 g/mole. Le bloc hydrosoluble neutre A' a une masse molaire allant de 500 g/mole à 250 000 g/mole, de préférence de 1 000 g/mole à 150 000 g/mole. Le bloc hydrophobe B a une masse molaire allant de 500 g/mole à 250 000 g/mole, de préférence de 1 000 g/mole à 150 000 g/mole.

La quantité du bloc hydrophile neutre A' dans le polymère diblocs A'-B est supérieure à 50 % du poids total du polymère diblocs et de préférence supérieure à 60 % du poids total du polymère diblocs A'-B.

Les compositions cosmétiques ou dermatologiques de l'invention peuvent présenter un pH variant dans une large gamme, et pouvant aller par exemple de 2 à 10, de préférence de 3 à 8 et mieux de 4 à 7.

La phase aqueuse des compositions selon l'invention comprend au moins de l'eau. Les compositions de l'invention peuvent contenir en plus de l'eau, une phase huileuse et/ou un ou plusieurs solvants organiques, hydrophiles, lipophiles et/ou amphiphiles, physiologiquement acceptables, c'est-à-dire bien tolérés et donnant un toucher cosmétiquement acceptable.

Les solvants organiques peuvent représenter de 5 à 50 % du poids total de la composition. Ils peuvent être choisis dans le groupe constitué par les solvants organiques hydrophiles, les solvants organiques lipophiles, les solvants amphiphiles, ou leurs mélanges. La quantité d'eau va de préférence de 10 à 99,99 % en poids par rapport au poids total de la composition.

Parmi les solvants organiques, on peut citer par exemple les mono-alcools inférieurs linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le glycérol, le sorbitol ; les mono- ou di-alkyle isosorbide dont les groupements alkyle ont de 1 à 5 atomes de carbone comme le diméthyl isosorbide ; les polyéthylène glycols notamment ceux ayant de 6 à 80 oxydes d'éthylène ; les éthers d'éthylène glycol comme le diéthylène glycol mono-méthyl ou mono-éthyl éther; les éthers de propylène glycol comme le dipropylène glycol méthyl éther ; les esters et éthers de polyol, tels que les esters de polypropylène glycol (PPG) et plus spécialement les esters de polypropylène glycol (PPG) et d'acide gras, les éthers de PPG et d'alcool gras comme le PPG-23 oléyl éther et le PPG-36 oléate ; les esters d'acide gras et d'alkyle, tels que l'adipate de diisopropyle, l'adipate de dioctyle, les benzoates d'alkyle ; et leurs mélanges.

La composition de l'invention peut comprendre au moins une phase grasse dite aussi phase huileuse.

La phase grasse ou phase huileuse des compositions selon l'invention représente de préférence de 0 % à 50 % en poids par rapport au poids total de la composition. Dans une émulsion, la phase huileuse représente de préférence de 0,1 à 50 % en poids et mieux de 0,5 à 30 % en poids par rapport au poids total de la composition.

La phase grasse ou phase huileuse contient habituellement au moins une huile. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R^{a}COOR^{b} et R^{a}OR^{b} dans laquelle R^{a} représente le reste d'un acide gras comportant de 8 à 29 atomes de carbone, et R^{b} représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12, le ceteareth-12 et le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

La composition de l'invention peut comprendre une phase aqueuse seule, ou une phase aqueuse et une phase grasse (émulsion E/H ou H/E) ou plusieurs phases aqueuses et une phase grasse (émulsion E/H/E) ou une phase aqueuse et plusieurs phases grasses (H/E/H). Elle peut ainsi constituer une solution, un gel, une émulsion (simple ou multiple).

Quand la composition est une émulsion, elle peut ne pas contenir de tensioactif émulsionnant mais elle peut aussi contenir au moins un tensioactif émulsionnant. Les tensioactifs émulsionnants sont choisis de manière appropriée suivant l'émulsion à obtenir (E/H ou H/E).

Pour les émulsions H/E, on peut utiliser par exemple comme tensioactif émulsionnant, un tensioactif émulsionnant non ionique, comme les esters et éthers d'oses tels que le stéarate de sucrose, le cocoate de sucrose, et le mélange de stéarate de sorbitan et de cocoate de sucrose commercialisé par la société ICI sous la dénomination d'Arlatone 2121®; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés tels que l'éther oxyéthyléné, oxypropyléné de l'alcool laurique à 25 groupes oxyéthylénés et 25 groupes oxypropylénés (nom CTFA « PPG-25 laureth-25 ») et l'éther oxyéthyléné du mélange d'alcools gras en C12-C15 comportant 7 groupes oxyéthylénés (nom CTFA « C12-C15 Pareth-7 ») ; les polymères d'éthylène glycol, tels que le PEG-100, et leurs mélanges.

Pour les émulsions E/H, on peut citer par exemple comme tensioactif émulsionnant, les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination PROTEGIN W® par la société Goldschmidt, l'isostéarate de sorbitan, le di-isostéarate de polyglycéryle, le sesqui-isostéarate de polyglycéryle-2 ; les esters et éthers d'oses tels que le « Methyl glucose dioleate » ; les esters gras tels que le lanolate de magnésium ; les dimethicone copolyols et alkyl-dimethicone copolyols tels que le Laurylmethicone copolyol vendu sous la dénomination DOW CORNING 5200 FORMULATION AID par la société Dow Corning et le Cetyl diméthicone copolyol vendu sous la dénomination ABIL EM 90® par la société Goldschmidt, et leurs mélanges.

Les tensioactifs émulsionnants peuvent être introduits tels quels ou sous forme de mélange avec d'autres tensioactifs émulsionnants et/ou avec d'autres composés tels que des alcools gras ou des huiles.

On peut aussi utiliser comme émulsionnants dans les émulsions E/H ou H/E, des polymères amphiphiles tels que les copolymères acryliques modifiés comme par exemple les produits commercialisés sous les dénominations Pemulen par la société Goodrich ; les copolymères d'acide 2-acrylamido-2-méthylpropane sulfonique à chaîne hydrophobe telsque décrits dans le document EP-1069142 ; les polyoléfines à terminaison succinique éventuellement estérifiée ou amidifiée, comme les composés décrits dans les documents US-A-4,234,435, US-A-4,708,753, US-A-5,129,972, US-A-4,931,110, GB-A-2,156,799 et US-A-4,919,179 incorporés ici pour référence. Comme polyoléfines à terminaison succinique, on peut citer notamment les polyisobutylènes à terminaison succinique modifiée, tels que les produits commercialisés sous les dénominations L2724 et L2721 par la société Lubrizol.

La quantité de tensioactif émulsionnant peut aller de 0 à 1 % dans les émulsions dites exemptes de tensioactif émulsionnant. Dans les autres émulsions, la quantité d'émulsionnants (tensioactifs émulsionnants ou polymères amphiphiles) peut aller de 0,01 à 10 % du poids total de la composition, et de préférence de 0,1 à 5 % en poids par rapport au poids total de la composition.

De façon connue, toutes les compositions de l'invention peuvent contenir des adjuvants habituels dans les domaines cosmétique et dermatologique, d'autres gélifiants et/ou épaississants classiques hydrophiles ou lipophiles ; des polymères ; des agents hydratants ; des tensioactifs moussants ; des émollients ; des filtres solaires ; des actifs hydrophiles ou lipophiles ; des agents anti-radicaux libres ; des séquestrants ; des antioxydants ; des conservateurs ; des agents alcanisants ou acidifiants ; des parfums ; des pigments ; des charges ; des agents filmogènes ; des matières colorantes, et leurs mélanges. Les quantités de ces différents adjuvants sont celles classiquement utilisées dans les domaines considérés.

### Gélifiants

Comme gélifiants, on peut citer par exemple les polymères hydrophiles tels que les polymères carboxyvinyliques comme les carbomers ; les polymères d'acide 2-acrylamido-2-méthylpropane sulfonique solubles ou dispersibles en phase aqueuse comme le polymère commercialisé sous la dénomination « Hostacerin AMPS » par la société Clariant ; les polymères neutres synthétiques comme le polyvinylpyrrolidone (PVP), l'acétate de polyvinyle (PVA) ; les polysaccharides comme les gommes de guar, de xanthane et les dérivés de cellulose comme par exemple l'hydroxyéthylcellulose ; les dérivés siliconés hydrosolubles ou hydrodispersibles comme les silicones acryliques et les silicones cationiques. On peut aussi utiliser des gélifiants lipophiles, tels que les argiles modifiés ou les polysaccharides modifiés.

### Actifs

Comme actifs utilisables dans la composition de l'invention, on peut citer par exemple les agents hydratants et par exemple les hydrolysats de protéines et les polyols tels que la glycérine, les glycols comme les polyéthylène glycols, et les dérivés de sucre ; les extraits naturels ; les anti-inflammatoires ; les oligomères procyannidoliques ; les vitamines comme la vitamine A (rétinol), la vitamine C (acide ascorbique), la vitamine E (tocophérol), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les enzymes ; la DHEA et ses dérivés et métabolites; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents tenseurs ; et leurs mélanges.

### Tensioactifs moussants

Comme tensioactifs moussants, on peut citer les tensioactifs moussants non ioniques, anioniques, amphotères et zwitterioniques et leurs mélanges.

Les tensioactifs moussants non ioniques peuvent être choisis par exemple parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges. Comme alkylpolyglucosides, on peut citer plus particulièrement le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP ® par la société Henkel, et le produit commercialisé sous la dénomination ORAMIX NS 10 ® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N ® et PLANTACARE 1200 ® par la société Henkel ; et le coco-glucoside comme le produit commercialisé sous la dénomination PLANTACARE 818/UP ® par la société Henkel.

Les tensioactifs anioniques peuvent être choisis notamment parmi les carboxylates ; les dérivés des aminoacides tels que les sarcosinates et notamment les acylsarcosinates comme le lauroyl sarcosinate de sodium, le myristoyl sarcosinate de sodium ; les alkyl sulfates ; les alkyl éther sulfates comme le lauryl éther sulfate de sodium et le lauryl éther sulfate d'ammonium ; les sulfonates comme par exemple les alpha-oléfines sulfonates ; les iséthionates et acyliséthionates comme le cocoyliséthionate de sodium ; les taurates ; les sulfosuccinates ; les alkyl sulfoacétates ; les phosphates et alkylphosphates comme le phosphate de lauryle ; les polypeptides ; les dérivés anioniques d'alkyl polyglucoside ; les savons d'acides gras comme les sels de potassium ou de sodium des acides laurique, myristique, palmitique, stéarique (laurate, myristate, palmitate et stéarate de potassium ou de sodium) ; et leurs mélanges.

Les tensioactifs amphotères et zwitterioniques peuvent être choisis par exemple parmi les bétaïnes comme la cocobétaïne, la laurylbétaïne, la laurylbétaïne oxyethylénée, la stéarylbétaïne oxyéthylénée ; les N-alkylamidobétaïnes comme la cocamidopropyl bétaine ; les dérivés de la glycine comme le N-cocoylglycinate de sodium ou de potassium ; les sultaïnes comme le cocoyl-amidopropylhydroxy-sulfobetaine ; les alkyl polyaminocarboxylates ; les alkylamphoacétates comme le N-cocoyl-N-carboxyméthoxyéthyl-N-carboxyméthyl-éthylènediamine N-di-sodique et le N-cocoyl-N-hydroxyéthyl-N-carboxyméthyl-éthylènediamine N-sodique ; et leurs mélanges.

### Filtres solaires

Les filtres solaires peuvent être choisis parmi les filtres organiques, les filtres physiques et leurs mélanges.

Comme exemples de filtres organiques actifs dans l'UV-A et/ou l'UV-B, on peut citer par exemple, désignés ci-dessus sous leur nom CTFA :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate vendu sous le nom « NEO HELIOPAN TS » par HAARMANN et REIMER,

### Dérivés du dibenzoylméthane:

- Butyl Methoxydibenzoylmethane vendu notamment sous le nom commercial « PARSOL 1789 » par HOFFMANN LA ROCHE,
- Isopropyl Dibenzoylmethane,

### Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivé du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX » par CHIMEX,
- Polyacrylamidomethyl Benzylidene Camphor fabriqué sous le nom « MESORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

### Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,

### Dérivés du phenyl benzotriazole :

- Drometrizole Trisiloxane vendu sous le nom « SILATRIZOLE » par RHODIA CHIMIE,

### Dérivés anthraniliques :

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés du benzalmalonate :

- Polyorganosiloxane à fonctions benzalmalonate vendu sous la dénomination commerciale « PARSOL SLX » par HOFFMANN LA ROCHE
et leurs mélanges.

Les filtres UV organiques plus particulièrement préférés sont choisis parmi les composés suivants
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- et leurs mélanges.

Comme filtres solaires physiques, on peut citer par exemple les oxydes de titane ou de zinc, sous forme de micro- ou nanoparticules (nanopigments) éventuellement enrobées, et leurs mélanges. On peut citer par exemple le nanotitane hydrophile commercialisé sous la dénomination MIRASUN TIW60 par la société Rhodia, et le nanotitane lipophile commercialisé sous la dénomination MT100T par la société TAYCA.

### Pigments

Les pigments sont notamment utilisés dans les compositions de maquillage. Comme pigments, on peut citer les pigments minéraux et notamment les oxydes métalliques tels que les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré ; et les pigments organiques tels que le noir de carbone et les laques qui sont des sels de calcium, de baryum, d'aluminium ou de zirconium, de colorants acides tels que les colorants halogéno-acides, azoïques ou anthraquinoniques.

Ces pigments peuvent être traités de manière à rendre leur surface hydrophobe; ce traitement peut être effectué selon les méthodes connues de l'homme du métier ; les pigments peuvent notamment être enrobés par des composés siliconés tels que des PDMS et/ou par des polymères.

### Charges

Comme charges, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gels, de lotions, de laits, de crèmes plus ou moins onctueuses, de pâtes. Ces compositions sont préparées selon les méthodes usuelles. Le polymère bloc utilisé étant hydrosoluble, il est généralement introduit dans une phase aqueuse.

Les compositions de l'invention peuvent être utilisées comme produit de soin, de traitement, de protection, de nettoyage, de démaquillage, et /ou de maquillage des matières kératiniques (peau, cheveux, cuir chevelu, cils, sourcils, ongles ou muqueuses) tels que des crèmes de protection, de traitement ou de soin pour le visage, pour les mains ou pour le corps, des laits corporels de protection ou de soin, des gels ou mousses pour le soin de la peau et/ou des muqueuses (lèvres).

Les compositions de l'invention peuvent contenir des filtres solaires et être ainsi également utilisées comme produit de protection solaire.

Les compositions peuvent être utilisées comme produits pour le maquillage, notamment le maquillage de la peau, des sourcils, des cils et des lèvres, tels que des crèmes pour le visage, des fonds de teint, des mascaras, des rouges à lèvres. De tels produits contiennent généralement des pigments.

Les compositions selon l'invention peuvent être également utilisées comme produits rincés ou comme produits non-rincés pour le nettoyage de la peau du visage et/ou du corps et/ou pour le nettoyage des cheveux, par exemple comme produits capillaires y compris pour le soin et le conditionnement des cheveux.

L'invention a pour objet l'utilisation cosmétique d'une composition cosmétique telle que définie ci-dessus, comme produit capillaire rincé ou non-rincé.

L'invention a pour objet l'utilisation cosmétique d'une composition cosmétique telle que définie ci-dessus, comme produit de nettoyage et/ou de démaquillage de la peau et/ou des yeux.

L'invention a aussi pour objet l'utilisation cosmétique d'une composition cosmétique telle que définie ci-dessus, comme produit de soin pour la peau, les cheveux, le cuir chevelu, des cils, des sourcils, des ongles ou des muqueuses.

L'invention a aussi pour objet l'utilisation cosmétique d'une composition cosmétique telle que définie ci-dessus, comme produit de maquillage.

L'invention a aussi pour objet l'utilisation cosmétique d'une composition cosmétique telle que définie ci-dessus, comme produit de protection solaire (protection contre le soleil et/ou les U.V. des appareils à bronzer).

Un autre objet de l'invention est un procédé de traitement cosmétique (non-thérapeutique) d'une matière kératinique (peau, cuir chevelu, cheveux, cils, sourcils, ongles ou muqueuses), caractérisé en ce qu'on applique sur la matière kératinique, une composition cosmétique telle que définie ci-dessus. La matière kératinique est notamment la peau.

### Exemples

Le pouvoir gélifiant des polymères a été mis en évidence par rhéologie à l'aide d'un rhéomètre de type RS150 (Haake) fonctionnant à contrainte imposée, et équipé d'une géométrie cône/plan 35mm/2°. Un système de régulation en température à effet Peltier permet d'assurer une mise en température de l'échantillon à 20°C pendant les mesures. Les caractérisations rhéologiques ont été réalisées en modes écoulement et dynamique.

### Mesures en écoulement :

Les mesures sont effectuées en imposant une rampe ascendante et une rampe descendante en contrainte à l'équilibre de 0 Pa jusqu'à une contrainte correspondant à une vitesse de cisaillement de 500 s⁻¹. Ces mesures permettent d'évaluer la viscosité des systèmes étudiés pour des vitesses de cisaillement égales à 0,01 s⁻¹ et 100 s⁻¹.

### Mesures en dynamique :

Les limites des domaines viscoélastiques linéaires ont été déterminées à 10⁻² et 1 Hz en soumettant l'échantillon à une série de contraintes sinusoïdales de fréquence fixe et d'amplitudes croissantes réparties de façon logarithmique entre deux bornes à raison de 5 points par décade. Le comportement viscoélastique linéaire est ensuite caractérisé en soumettant l'échantillon à une série de 20 contraintes sinusoïdales de fréquences logarithmiquement réparties entre 10 et 10⁻² Hz et d'amplitudes telles que l'amplitude de déformation soit constante et située dans le domaine linéaire précédemment déterminé. Ces mesures permettent d'évaluer le module complexe G* à 1 Hz et l'angle de perte δ à 1 Hz, dans le domaine de viscoélasticité linéaire. G* et δ sont les paramètres viscoélastiques utilisés pour mesurer les propriétés physiques des fluides viscoélastiques, comme expliqué dans « An introduction to rheology » de H.A BARNES, J.F HUTTON, K. WALTERS, pages 46 à 54 (Ed. Elsevier 1989). Le module G* est égale à la racine carré de la somme des carrés de modules élastique G' et de perte G". La tangente de l'angle de perte δ est égale au rapport G"/G'.

Dans les exemples suivants, le polymère diblocs utilisé a été préparé par polymérisation anionique.

### Exemple 1 : Solution aqueuse contenant 3% (en poids) d'un polymère diblocs Polystyrène (2000 g/mole) - Polyacrylate de sodium (11500 g/mole). Dans ce polymère diblocs, le bloc ionique hydrosoluble (Polyacrylate de sodium) représente 85,2 % du poids total du polymère diblocs.

Cette solution est préparée par simple dissolution de la quantité adéquate de polymère sous forme poudre dans l'eau déminéralisée, sous agitation à 25°C. La préparation de cette solution ne nécessite pas de procédé de dispersion spécifique. Cette solution est transparente et gélifiée.

### Mesures rhéologiques en écoulement :

Viscosité (0,01 s⁻¹) = 300 Pa.s
Viscosité (100 s⁻¹) = 0,4 Pa.s

### Mesures rhéologiques en dynamique :

G*(1 Hz) = 80 Pa
δ (1 Hz) = 7°

Le polymère diblocs Polystyrène (2000 g/mole) - Polyacrylate de sodium (11500 g/mole) présente un pouvoir gélifiant de l'eau à une faible concentration massique (3 %). Cette solution présente un caractère rhéofluidifiant et un comportement élastique marqués. Le pouvoir gélifiant de ce polymère est reproductible entre différents lots.

**Exemple 1 comparatif:** Solution aqueuse contenant 3% (en poids) d'un polymère diblocs Polystyrène (5100 g/mole) - Polyacrylate de sodium (5060 g/mole). Dans ce polymère diblocs, le bloc ionique hydrosoluble (Polyacrylate de sodium) représente 49,8 % du poids total du polymère diblocs.

Cette solution est préparée par simple dissolution de la quantité adéquate de polymère sous forme poudre dans l'eau déminéralisée, sous agitation à 25°C. Cette solution est trouble et fluide.

### Mesures rhéologiques en écoulement :

Viscosité (0,01 s⁻¹) = 0,01 Pa.s
Viscosité (100 s⁻¹) = 0,01 Pa.s

Le polymère diblocs Polystyrène (5100 g/mole) - Polyacrylate de sodium (5060 g/mole) n'est que partiellement soluble dans l'eau à une faible concentration massique (3 %) et ne présente pas de pouvoir gélifiant de l'eau dans ces conditions de concentrations.

### Exemple 2 : Solution aqueuse contenant 1,5 % en poids, d'un polymère diblocs Polystyrène (2000 g/mole) - Polyacrylate de sodium (11500 g/mole) et 1,5 % en poids, d'un polymère diblocs Polystyrène (3600 g/mole) - Poly-oxyde d'éthylène (7000 g/mole).

Cette solution est préparée par dissolution de la quantité indiquée de polymères sous forme poudre dans l'eau déminéralisée, sous agitation à 25°C. La solution obtenue est transparente et gélifiée.

### Mesures rhéologiques en écoulement :

Viscosité (0,01 s⁻¹) = 300 Pa.s
Viscosité (100 s⁻¹) = 0,3 Pa.s

### Mesures rhéologiques en dynamique :

G*(1 Hz) = 70 Pa
δ (1 Hz) = 12°

Le mélange de 1,5 % d'un polymère diblocs Polystyrène (2000 g/mole) - Polyacrylate de sodium (11500 g/mole) et 1,5 % en poids, d'un polymère diblocs Polystyrène (3600 g/mole) - Poly-oxyde d'éthylène (7000 g/mole) présente un pouvoir gélifiant de l'eau à une faible concentration massique (3 %). La solution aqueuse obtenue présente un caractère rhéofluidifiant et un comportement élastique marqués. Le pouvoir gélifiant de ce mélange est reproductible entre différents lots.

Pour comparaison, une solution aqueuse contenant 1,5 % en poids, d'un polymère diblocs Polystyrène (3600 g/mole) - Poly-oxyde d'éthylène (7000 g/mole) a été préparée par dissolution de la quantité adéquate de polymères sous forme poudre dans l'eau déminéralisée, sous agitation à 25°C. La solution obtenue est transparente et fluide.

### Mesures rhéologiques en écoulement :

Viscosité (0,01 s⁻¹) = 0,013 Pa.s
Viscosité (100 s⁻¹) = 0,010 Pa.s

Le polymère diblocs Polystyrène (3600 g/mole) - Poly-oxyde d'éthylène (7000 g/mole) est soluble dans l'eau à une concentration en poids de 3 % mais ne présente pas de pouvoir gélifiant de l'eau dans ces conditions de concentrations.

Par ailleurs, on a préparé une solution aqueuse contenant 1,5 % en poids, d'un polymère diblocs Polystyrène (2000 g/mole) - Polyacrylate de sodium (11500 g/mole), par dissolution de la quantité indiquée de polymère sous forme poudre dans l'eau déminéralisée, sous agitation à 25°C. La solution obtenue est transparente et gélifiée.

### Mesures rhéologiques en écoulement :

Viscosité (0,01 s⁻¹) = 7 Pa.s
Viscosité (100 s⁻¹) = 0,03 Pa.s

### Mesures rhéologiques en dynamique :

G*(1 Hz) =1,1 Pa
δ (1 Hz) = 70°

Ces résultats montrent que l'association du polymère diblocs Polystyrène (2000 g/mole) - Polyacrylate de sodium (11500 g/mole) et du polymère diblocs Polystyrène (3600 g/mole) - Poly-oxyde d'éthylène (7000 g/mole) permet d'obtenir une solution aqueuse gélifiée à de faibles concentrations en poids (1,5 % en poids de chaque polymère). Les propriétés de gélification observées sont très supérieures à celles des solutions à 1,5 % de chacun des polymères diblocs ioniques et neutres considérés séparément. Ces résultats mettent en évidence un effet de synergie de gélification pour le mélange des polymères diblocs ionique A-B et neutre A'-B.

### Exemple 3 : Solution aqueuse contenant 1% (en poids) d'un copolymère diblocs Polystyrène (1 800 g/mole)- Polyacrylate de sodium (42 450 g/mole). Dans ce polymère diblocs, le bloc ionique hydrosoluble (Polyacrylate de sodium) représente 95,9% du poids total du polymère diblocs.

Cette solution est préparée par simple dissolution de la quantité adéquate de polymère sous forme poudre dans l'eau déminéralisée, sous agitation à 50°C pendant 7 heures. Après refroidissement à 25°C, cette solution est gélifiée et transparente.

### Mesures rhéologiques en dynamique :

Ces mesures sont réalisées à l'aide d'un rhéomètre Rheometrics RFS III muni d'une géométrie cône plan 25 mm/0,04 rad, dans les conditions de mesures suivantes :
- Déformation = 1 % afin de se situer dans le domaine de viscoélasticité linéaire,
- Fréquences comprises entre 0,01 rad/s et 100 rad/s,

Les valeurs des modules élastique G' et visqueux G" mesurées pour une fréquence égale à 1 rad/s sont les suivantes :
G' (1 rad/s) = 250 Pa
G" (1 rad/s) = 20 Pa

La solution gélifiée à l'aide de 1% (en poids) de polymère diblocs Polystyrène (1 800 g/mole)- Polyacrylate de sodium (42 450 g/mole) présente un module élastique G' supérieur au module visqueux G". Cet exemple montre que l'on obtient une bonne gélification même pour de faibles concentrations de polymère.

### Exemple 4 : Solution aqueuse contenant 5 % (en poids) d'un polymère diblocs Polystyrène (2000 g/mole) - Polyacrylate de sodium (11500 g/mole). Dans ce polymère diblocs, le bloc ionique hydrosoluble (Polyacrylate de sodium) représente 85,2 % du poids total du polymère diblocs (copolymère diblocs de l'exemple 1).

Cette solution est préparée par simple dissolution de la quantité adéquate de polymère sous forme poudre dans l'eau déminéralisée, sous agitation à 50°C pendant 7 heures. Après refroidissement à 25°C, cette solution est gélifiée et transparente.

### Mesures rhéologiques en dynamique :

Ces mesures sont réalisées à l'aide d'un rhéomètre Rheometrics RFS III muni d'une géométrie cône plan 25 mm/0,04 rad, dans les conditions de mesures suivantes :
- Déformation = 1 % afin de se situer dans le domaine de viscoélasticité linéaire,
- Fréquences comprises entre 0,01 rad/s et 100 rad/s,

Les valeurs des modules élastique G' et visqueux G" mesurées pour une fréquence égale à 1 rad/s sont les suivantes :
G' (1 rad/s) = 2000 Pa
G" (1 rad/s) = 100 Pa

### Exemple 5 : influence des tensioactifs sur le pouvoir gélifiant

Cet exemple met en évidence le maintien du pouvoir gélifiant des polymères utilisés selon l'invention, en présence de tensioactifs.

On prépare par simple dissolution de la quantité adéquate de polymère et de tensioactif sous forme poudre dans l'eau déminéralisée, sous agitation à 25°C, une solution aqueuse contenant 3 % (en poids) d'un polymère diblocs Polystyrène (2000 g/mole) - Polyacrylate de sodium (11500 g/mole). Cette solution est transparente et gélifiée.

### Mesures rhéologiques en écoulement :

Sans tensioactif :
Viscosité (0,01 s⁻¹) = 300 Pa.s
Viscosité (100 s⁻¹) = 0,4 Pa.s

Avec 3 % de tensioactif non ionique (PEG-100 stéarate) :
Viscosité (0,01 s⁻¹) = 500 Pa.s
Viscosité (100 s⁻¹) = 0,5 Pa.s

Le pouvoir gélifiant n'est pas affecté par la présence de tensioactif.

### Exemple 6 : Sérum anti-âge

| | |
|---|---|
| Polymère diblocs Polystyrène (2000 g/mole) - | |
| Polyacrylate de sodium (11500 g/mole) | 2 % |
| Conservateur | 0,2 % |
| Acide ascorbique | 10 % |
| Dipropylène glycol | 5 % |
| Eau déminéralisée | 82,8 % |

Le sérum est préparé par dissolution du polymère diblocs dans de l'eau déminéralisée contenant le conservateur, l'acide ascorbique et le dipropylène glycol, sous agitation pendant 2 heures.

Le polymère diblocs Polystyrène (2000 g/mole) - Polyacrylate de sodium (11500 g/mole) permet à lui seul d'épaissir la phase aqueuse. La formule obtenue est un sérum anti-âge ayant une belle texture.

### Exemple 7 : Crème pour le corps

| *Phase aqueuse :* | |
|---|---|
| Polymère diblocs Polystyrène (2000 g/mole) - Polyacrylate de sodium (11500 g/mole) | 2,6 % |
| Conservateur | 0,2 % |
| Eau déminéralisée | 82,2 % |

| *Phase huileuse* | |
|---|---|
| Huile de Parléam | 9 % |
| Cyclohexadiméthylsiloxane | 6 % |

### Mode opératoire : La phase aqueuse est préparée par dissolution du polymère dans de l'eau déminéralisée contenant le conservateur, sous agitation pendant 2 heures. La phase huileuse est alors introduite lentement dans la phase aqueuse sous agitation.

Le polymère diblocs Polystyrène (2000 g/mole) - Polyacrylate de sodium (11500 g/mole) permet à lui seul de gélifier la phase aqueuse et d'émulsionner la totalité de la phase huileuse. On obtient une belle émulsion gélifiée pouvant être utilisée comme crème pour le corps.

## Revendications

1. Composition cosmétique et/ou dermatologique, **caractérisée en ce qu**'elle comprend au moins une phase aqueuse comportant au moins un polymère hydrosoluble ou hydrodispersible, de structure diblocs A-B où A est un bloc polymérique hydrosoluble ionique et B un bloc polymérique hydrophobe, la quantité de bloc polymérique A étant égale ou supérieure à 60 % du poids total du polymère diblocs, et en ce qu'elle comprend au moins une phase huileuse.

2. Composition selon la revendication 1, **caractérisée en ce que** la quantité de bloc polymérique A est égale ou supérieure à 70 % du poids total du polymère diblocs.

3. Composition selon la revendication 1 ou 2, **caractérisée en ce que** le bloc hydrosoluble ionique A est obtenu à partir d'un ou plusieurs monomères hydrosolubles (la) ou de leurs sels, les monomères (la) étant choisis parmi :
- l'acide (méth)acrylique,
- l'acide styrène sulfonique,
- l'acide vinylsulfonique et l'acide (méth)allylsulfonique,
- l'acide vinyl phosphonique,
- l'acide maléique,
- l'acide itaconique,
- l'acide crotonique,
- le chlorure de diméthyldiallyl ammonium,
- le chlorure de méthylvinylimidazolium,
- les carboxybétaïnes ou sulfobétaïnes éthyléniques,
- les monomères vinyliques hydrosolubles de formule (I) suivante : 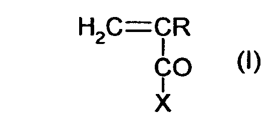
dans laquelle :
- R est choisi parmi H, -CH₃, -C₂H₅ ou -C₃H₇ ;
- X est choisi parmi :
- les oxydes d'alkyle de type -OR₁ où R₁ est un radical hydrocarboné, linéaire ou ramifié, saturé ou insaturé, ayant de 1 à 6 atomes de carbone, substitué par au moins un groupement carboxylate ; sulfonique ; sulfate ; phosphate et/ou ammonium quaternaire (-N⁺R₂R₃R₄), les radicaux R₂, R₃ et R₄ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₂ + R₃ + R₄ ne dépasse pas 6 ; le radical R₁ étant éventuellement substitué par un atome d'halogène ; un groupement hydroxy ; acide carboxylique ; éther ; amine primaire ; amine secondaire (-NHR₅) ; ou amine tertiaire (-NR₅R₆), les radicaux R₅ et R₆ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₅ + R₆ ne dépasse pas 6 ;
- les groupements -NH₂, -NHR₇ et -NR₇R₈ dans lesquels R₇ et R₈ sont, indépendamment l'un de l'autre, un radical hydrocarboné, linéaire ou ramifié, saturé ou insaturé ayant 1 à 6 atomes de carbone, sous réserve que le nombre total d'atomes de carbone de R₇ + R₈ ne dépasse pas 6, lesdits radicaux R₇ et/ou R₈ étant substitués par au moins un groupement carboxylate ; sulfonique; sulfate ; phosphate et/ou amine quaternaire (-N⁺R₉R₁₀R₁₁), les radicaux R₉, R₁₀ et R₁₁ étant, indépendamment l'un de l'autre, un radical hydrocarboné, linéaire ou ramifié, saturé ou insaturé ayant 1 à 6 atomes de carbone, sous réserve que la somme des atomes de carbone de R₇ + R₈ + R₉ + R₁₀ + R₁₁ ne dépasse pas 6; les radicaux R₇ et/ou R₈ étant éventuellement substitués par un atome d'halogène ; ou un groupement hydroxy ; acide carboxylique ; éther ; amine primaire ; amine secondaire (-NHR₅) ; ou amine tertiaire (-NR₅R₆), où R₅ et R₆ ont les significations indiquées ci-dessus, sous réserve que la somme des atomes de carbone de R₇+ R₈ + R₅ + R₆ ne dépasse pas 6 ;
- les groupes R et X étant tels que le monomère de formule (I) soit hydrosoluble ;
- les mélanges de ces monomères.

4. Composition selon la revendication précédente, **caractérisée en ce que** le bloc hydrosoluble ionique A est obtenu en outre à partir d'un ou plusieurs monomères choisis parmi les monomères hydrophobes (Ib), les monomères hydrosolubles neutres (Ic), et leurs mélanges.

5. Composition selon la revendication précédente, **caractérisée en ce que** le monomère hydrophobe (Ib) est choisi parmi :
- le styrène et ses dérivés ;
- l'acétate de vinyle ;
- les vinyléthers de formule CH₂=CHOR₁₂ dans laquelle R₁₂ est un radical hydrocarboné, linéaire ou ramifié, saturé ou insaturé, ayant de 1 à 6 atomes de carbone ;
- l'acrylonitrile ;
- la caprolactone ;
- le chlorure de vinyle et le chlorure de vinylidène ;
- les monomères siliconés insaturés ;
- les monomères vinyliques hydrophobes de formule (II) suivante : 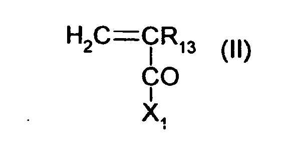
dans laquelle :
- R₁₃ est choisi parmi H, -CH₃, -C₂H₅ ou -C₃H₇ ;
- X₁ est choisi parmi :
- les oxydes d'alkyle de type -OR₁₄ où R₁₄ est un radical hydrocarboné, linéaire ou ramifié, saturé ou insaturé, ayant de 1 à 6 atomes de carbone ;
- les groupements -NH₂, -NHR₁₅ et -NR₁₅R₁₆ dans lesquels R₁₅ et R₁₆ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₅ + R₁₆ ne dépasse pas 6 ;
- les substituants R₁₃ et X₁ étant tels que le monomère de formule (II) soit hydrophobe ;
- les mélanges de ces monomères.

6. Composition selon la revendication 4 ou 5, **caractérisée en ce que** le monomère neutre (Ic) est choisi parmi :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyle cyclique ayant de 4 à 9 atomes de carbone,
- l'alcool vinylique,
- les monomères vinyliques hydrosolubles de formule (III) suivante : 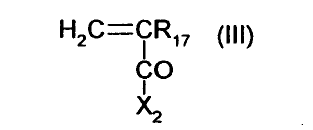
dans laquelle :
- R₁₇ est choisi parmi H, -CH₃, -C₂H₅ ou -C₃H₇
- X₂ est choisi parmi :
- les oxydes d'alkyle de type -OR₁₈ où R₁₈ est un radical hydrocarboné, linéaire ou ramifié, saturé ou insaturé, ayant de 1 à 6 atomes de carbone, éventuellement substitué par un atome d'halogène ; un groupement hydroxy ; acide carboxylique ; éther ; amine primaire ; amine secondaire (-NHR₁₉), ou amine tertiaire (-NR₁₉R₂₀) ; les radicaux R₁₉ et R₂₀ étant, indépendamment l'un de l'autre, un radical hydrocarboné, linéaire ou ramifié, saturé ou insaturé ayant 1 à 6 atomes de carbone, sous réserve que la somme des atomes de carbone de R₁₈ + R₁₉ + R₂₀ ne dépasse pas 6 ;
- les groupements -NH₂, -NHR₂₁ et -NR₂₁R₂₂ dans lesquels R₂₁ et R₂₂ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₂₁ + R₂₂ ne dépasse pas 6, lesdits radicaux R₂₁ et R₂₂ étant éventuellement substitués par un atome d'halogène ; un groupement acide carboxylique ; hydroxy ; éther; amine primaire ; amine secondaire (-NHR₂₃), tertiaire (-NR₂₃R₂₄), les radicaux R₂₃ et R₂₄ étant, indépendamment l'un de l'autre, un radical hydrocarboné, linéaire ou ramifié, saturé ou insaturé ayant 1 à 6 atomes de carbone, sous réserve que la somme des atomes de carbone de R₂₁ + R₂₂ + R₂₃ + R₂₄ ne dépasse pas 6 ;
- les substituants R₁₇ et X₂ étant tels que le monomère de formule (III) soit hydrosoluble ;
- les mélanges de ces monomères.

7. Composition selon la revendication 1, **caractérisée en ce que** le bloc hydrosoluble ionique A est la polyéthylène imine.

8. Composition selon l'une quelconque des revendications précédentes, **caractérisée en ce que** le bloc hydrosoluble ionique A est totalement ou partiellement neutralisé par une base minérale ou organique.

9. Composition selon l'une quelconque des revendications précédentes, **caractérisée en ce que** le bloc hydrophobe B est obtenu à partir d'un ou plusieurs monomères hydrophobes (Id) choisis parmi :
- le styrène et ses dérivés,
- l'acétate de vinyle,
- les vinyléthers de formule CH₂=CHOR₂₅ dans laquelle R₂₅ est un radical hydrocarboné, linéaire ou ramifié, saturé ou insaturé, ayant de 1 à 22 atomes de carbone,
- l'acrylonitrile,
- le chlorure de vinyle et le chlorure de vinylidène,
- la caprolactone,
- les alcènes,
- les monomères siliconés insaturés,
- les dérivés siliconés,
- les monomères vinyliques hydrophobes de formule (IV) suivante : 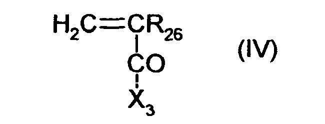
dans laquelle :
- R₂₆ est choisi parmi H, -CH₃, -C₂H₅ ou -C₃H₇;
- X₃ est choisi parmi :
- les oxydes d'alkyle de type -OR₂₇ où R₂₇ est un radical hydrocarboné, linéaire ou ramifié, saturé ou insaturé, ayant de 1 à 22 carbones, éventuellement substitué par un atome d'halogène ; un groupement carboxylate ; sulfonique ; sulfate ; phosphate ; hydroxy ; acide carboxylique ; éther ; amine primaire ; amine secondaire (-NHR₂₈) ; amine tertiaire (-NR₂₈R₂₉) ou ammonium quaternaire (-N⁺R₂₈R₂₉R₃₀), les radicaux R₂₈, R₂₉ et R₃₀ étant, indépendamment l'un de l'autre, un radical hydrocarboné, linéaire ou ramifié, saturé ou insaturé ayant 1 à 22 atomes de carbone, sous réserve que la somme des atomes de carbone de R₂₇ + R₂₈ + R₂₉ + R₃₀ ne dépasse pas 22 ; ou bien R₂₇ est un radical perfluoroalkyle, comportant de 1 à 18 atomes de carbone ;
- les groupements -NH₂, -NHR₃₁ et -NR₃₁R₃₂ dans lesquels les radicaux R₃₁ et R₃₂ sont, indépendamment l'un de l'autre, un radical hydrocarboné, linéaire ou ramifié, saturé ou insaturé ayant 1 à 22 atomes de carbone, sous réserve que le nombre total d'atomes de carbone de R₃₁ + R₃₂ ne dépasse pas 22, lesdits radicaux R₃₁ et R₃₂ étant éventuellement substitués par un atome d'halogène ; un groupement hydroxy ; éther ; carboxylate ; sulfonique ; sulfate ; phosphate ; acide carboxylique ; amine primaire ; amine secondaire (-NHR₂₈) ; amine tertiaire (-NR₂₈R₂₉) ou ammonium quaternaire (-N⁺R₂₈R₂₉R₃₀) où R₂₈, R₂₉ et R₃₀ ont les mêmes significations que celles indiquées ci-dessus, sous réserve que la somme des atomes de carbone de R₃₁ + R₃₂ + R₂₈ + R₂₉ + R₃₀ ne dépasse pas 22 ; ou bien R₃₁ et R₃₂ indépendamment l'un de l'autre sont un radical perfluoroalkyle comportant de 1 à 18 atomes de carbone ;
- les substituants R₂₆ et X₃ étant tels que le monomère de formule (IV) soit hydrophobe ;
- et les mélanges de ces monomères.

10. Composition selon la revendication précédente, **caractérisée en ce que** le bloc hydrophobe B est en outre obtenu à partir d'un ou plusieurs monomères hydrosolubles ioniques ou neutres (le) choisis parmi les monomères suivants ou leurs sels :
- l'acide (méth)acrytique ;
- l'acide styrène sulfonique ;
- l'acide vinylsulfonique et l'acide (méth)allylsulfonique ;
- l'acide vinyl phosphonique ;
- l'anhydride maléique ;
- l'acide maléique ;
- l'acide itaconique ;
- l'acide crotonique ;
- le chlorure de diméthyldiallyl ammonium ;
- le chlorure de méthylvinylimidazolium ;
- le (méth)acrylamide ;
- la N-vinylacétamide et la N-méthyl N-vinylacétamide;
- la N-vinylformamide et la N-méthyl N-vinylformamide ;
- les N-vinyllactames comportant un groupe alkyle cyclique ayant de 4 à 9 atomes de carbone,
- l'alcool vinylique ;
- la 2-vinylpyridine et la 4-vinylpyridine ;
- les monomères vinyliques hydrosolubles de formule (V) suivante : 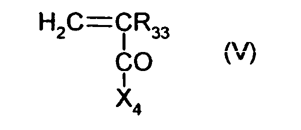
dans laquelle :
- R₃₃ est choisi parmi H, -CH₃, -C₂H₅ ou -C₃H₇ ;
- X₄ est choisi parmi :
- les oxydes d'alkyle de type -OR₃₄ où R₃₄ est un radical hydrocarboné, linéaire ou ramifié, saturé ou insaturé, ayant de 1 à 6 atomes de carbone, éventuellement substitué par un atome d'halogène ; un groupement carboxylate ; acide carboxylique ; sulfonique ; sulfate ; phosphate ; hydroxy ; éther ; amine primaire ; amine secondaire (-NHR₃₅) ; amine tertiaire (-NR₃₅R₃₆); ou ammonium quaternaire (-N⁺R₃₅R₃₆R₃₇), les radicaux R₃₅, R₃₆ et R₃₇ étant, indépendamment l'un de l'autre, un radical hydrocarboné, linéaire ou ramifié, saturé ou insaturé ayant 1 à 6 atomes de carbone, sous réserve que la somme des atomes de carbone de R₃₄ + R₃₅ + R₃₆ + R₃₇ ne dépasse pas 6 ;
- les groupements -NH₂, -NHR₃₈ et -NR₃₈R₃₉ dans lesquels les radicaux R₃₈ et R₃₉ sont, indépendamment l'un de l'autre, des radicaux hydrocarbonés, linéaires ou ramifiés, saturés ou insaturés ayant 1 à 6 atomes de carbone, sous réserve que le nombre total d'atomes de carbone de R₃₈ + R₃₉ ne dépasse pas 6, lesdits radicaux R₃₈ et/ou R₃₉ étant éventuellement substitués par un atome d'halogène ; un groupement carboxylate ; acide carboxylique ; sulfonique ; sulfate ; phosphate ; hydroxy ; éther ; amine primaire ; amine secondaire (-NHR₃₅) ; amine tertiaire (-NR₃₅R₃₆) ou ammonium quaternaire (-N⁺R₃₅R₃₆R₃₇) où R₃₅, R₃₆ et R₃₇ ont la même signification qu'indiquée ci-dessus, sous réserve que la somme des atomes de carbone de R₃₈ + R₃₉ + R₃₅ + R₃₆ + R₃₇ ne dépasse pas 6 ;
- les substituants R₃₃ et X₄ étant tels que le monomère de formule (V) soit hydrosoluble ;
- et les mélanges de ces monomères.

11. Composition selon l'une quelconque des revendications précédentes, **caractérisée en ce que** le polymère diblocs a une masse molaire allant de 1 000 g/mole à 500 000 g/mole.

12. Composition selon l'une quelconque des revendications précédentes, **caractérisée en ce que** le polymère diblocs a une masse molaire allant de 2 000 g/mole à 300 000 g/mole.

13. Composition selon l'une quelconque des revendications 1 à 3, 7 à 9 et 11 à 12, **caractérisée en ce que** le bloc polymérique ionique A est totalement hydrosoluble et le bloc polymérique B est totalement hydrophobe.

14. Composition selon la revendication précédente, **caractérisée en ce que** le polymère diblocs comporte, comme bloc A, du polyacrylate de sodium et, comme bloc B, du polystyrène.

15. Composition selon l'une quelconque des revendications précédentes, **caractérisée en ce que** la quantité de polymère(s) diblocs va de 0,01 à 20 % en poids par rapport au poids total de la composition.

16. Composition selon l'une quelconque des revendications précédentes, **caractérisée en ce qu**'elle contient en outre au moins un polymère hydrosoluble ou hydrodispersible diblocs neutre A'-B dans lequel A' est un bloc polymérique hydrosoluble neutre et B un bloc polymérique hydrophobe conforme aux revendications 9 et 10.

17. Composition selon la revendication précédente, **caractérisée en ce que** la quantité du polymère diblocs ionique A-B est supérieure à 10 % en poids par rapport à la quantité totale de polymères A-B et A'-B.

18. Composition selon la revendication 16 ou 17, **caractérisée en ce que** le bloc A' est obtenu à partir d'un ou plusieurs monomères hydrosolubles choisis parmi les monomères (If) suivants :
- le (méth)acrylamide,
- la N-vinylacétamide et la N-méthyl N-vinylacétamide,
- la N-vinylformamide et la N-méthyl N-vinylformamide,
- les N-vinyllactames comportant un groupe alkyl cyclique ayant de 4 à 9 atomes de carbone,
- l'alcool vinylique,
- l'anhydride maléique,
- la vinylamine,
- les monomères vinyliques hydrosolubles de formule (VI) suivante : 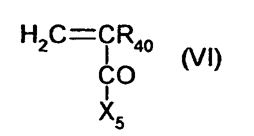
dans laquelle :
- R₄₀ est choisi parmi H, -CH₃, -C₂H₅ ou -C₃H₇ ;
- X₅ est choisi parmi :
- les oxydes d'alkyle de type -OR₄₁ où R₄₁ est un radical hydrocarboné, linéaire ou ramifié, saturé ou insaturé, ayant de 1 à 6 atomes de carbone, éventuellement substitué par un atome d'halogène ; un groupement acide carboxylique ; hydroxy ; éther ; amine primaire ; amine secondaire (-NHR₄₂) ; ou amine tertiaire (-NR₄₂R₄₃) ; les radicaux R₄₂ et R₄₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄₁ + R₄₂ + R₄₃ ne dépasse pas 6 ;
- les groupements -NH₂, -NHR₄₄ et -NR₄₄R₄₅ dans lesquels R₄₄ et R₄₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄₄ + R₄₅ ne dépasse pas 6, lesdits radicaux R₄₄ et R₄₅ étant éventuellement substitués par un atome d'halogène ; un groupement acide carboxylique ; hydroxy; éther; amine primaire ; amine secondaire (-NHR₄₆); ou amine tertiaire (-NR₄₆R₄₇), les radicaux R₄₆ et R₄₇ étant, indépendamment l'un de l'autre, un radical hydrocarboné, linéaire ou ramifié, saturé ou insaturé ayant 1 à 6 atomes de carbone, sous réserve que la somme des atomes de carbone de R₄₄ + R₄₅ + R₄₆ et R₄₇ ne dépasse pas 6 ;
- les substituants R₄₀ et X₅ étant tels que le monomère de formule (VI) soit hydrosoluble ;
- et les mélanges de ces monomères.

19. Composition selon la revendication précédente, **caractérisée en ce que** le bloc hydrosoluble neutre A' est obtenu en outre à partir d'un ou plusieurs monomères hydrophobes (Ig) choisi parmi les monomères suivants :
- le styrène et ses dérivés,
- l'acétate de vinyle,
- les vinyléthers de formule CH₂=CHOR₄₈ dans laquelle R₄₈ est un radical hydrocarboné, linéaire ou ramifié, saturé ou insaturé, ayant de 1 à 6 atomes de carbone,
- l'acrylonitrile,
- la caprolactone,
- le chlorure de vinyle et le chlorure de vinylidène,
- les monomères siliconés insaturés,
- les monomères vinyliques hydrophobes de formule (VII) suivante : 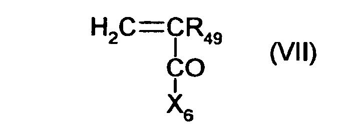
dans laquelle :
- R₄₉ est choisi parmi H, -CH₃, -C₂H₅ ou -C₃H₇;
- X₆ est choisi parmi :
- les oxydes d'alkyle de type -OR₅₀ où R₅₀ est un radical hydrocarboné, linéaire ou ramifié, saturé ou insaturé, ayant de 1 à 6 atomes de carbone ;
- les groupements -NH₂, -NHR₅, et -NR₅₁R₅₂ dans lesquels R₅₁ et R₅₂ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₅₁ + R₅₂ ne dépasse pas 6 ;
- les substituants R₄₉ et X₆ étant tels que le monomère de formule (VII) soit hydrophobe ;
- et les mélanges de ces monomères.

20. Composition selon l'une quelconque des revendications 16 à 19, **caractérisée en ce que** le polymère diblocs neutre a une masse molaire allant de 1 000 g/mole à 500 000 g/mole.

21. Composition selon l'une quelconque des revendications 16 à 20, **caractérisée en ce que** la quantité du bloc hydrophile neutre A dans !e polymère diblocs A'-B est supérieure à 50 % du poids total du polymère diblocs.

22. Composition selon l'une quelconque des revendications précédentes,
**caractérisée en ce qu**'elle contient un milieu physiologiquement acceptable.

23. Composition selon l'une quelconque des revendications précédentes,
**caractérisée en ce qu**'elle contient en outre au moins un solvant organique choisi dans le groupe constitué par les solvants organiques hydrophiles, les solvants organiques lipophiles, les solvants amphiphiles ou leurs mélanges.

24. Composition selon la revendication précédente, **caractérisée en ce que** les solvants organiques sont choisis dans le groupe constitué par les mono-alcools ayant de 1 à 8 atomes de carbone, les polyols, les mono- et di-alkyl isosorbides, les polyéthylène glycols, les éthers d'éthylène glycol, les éthers de propylène glycol, les éthers de polyol, les esters de polyol, les esters d'acide gras et d'alkyle, et leurs mélanges.

25. Composition selon la revendication 23 ou 24, **caractérisée en ce que** le ou les solvants organiques représentent de 5 à 50 % du poids total de la composition.

26. Composition selon l'une quelconques des revendications précédentes, **caractérisée en ce que** la phase huileuse comprend au moins une huile.

27. Composition selon l'une quelconque des revendications précédentes, **caractérisée en ce qu**'elle constitue une émulsion H/E, E/H, E/H/E, H/E/H.

28. Composition selon l'une quelconque des revendications précédentes, **caractérisée en ce qu**'elle contient en outre au moins un adjuvant choisi dans le groupe constitué par les gélifiants et/ou épaississants ; les polymères ; les tensioactifs moussants ; les agents hydratants ; les émollients ; les actifs hydrophiles ou lipophiles ; les agents anti-radicaux libres ; les séquestrants ; les antioxydants ; les conservateurs ; les agents alcanisants ou acidifiants ; les parfums ; les pigments ; les charges ; les agents filmogènes; les matières colorantes ; et leurs mélanges.

29. Composition selon la revendication précédente, **caractérisée en ce que** l'actif est choisi parmi les hydrolysats de protéines ; les anti-inflammatoires ; les polyols ; les dérivés de sucre ; les extraits naturels; les oligomères procyannidoliques; les vitamines ; l'urée; la caféine; les dépigmentants ; l'acide salicylique et ses dérivés ; les alpha-hydroxyacides; les rétinoïdes ; les filtres solaires ; l'hydrocortisone; la mélatonine ; les extraits d'algues, de champignons, de végétaux, de levures, de bactéries ; les enzymes ; la DHEA et ses dérivés et métabolites ; les actifs anti-bactériens ; les agents matifiants ; les agents tenseurs ; et leurs mélanges.

30. Composition selon la revendication précédente, **caractérisée en ce que** l'actif est choisi parmi la vitamine A, la vitamine C, la vitamine E, la vitamine B5, la vitamine B3, leurs esters et leurs mélanges.

31. Composition selon la revendication 29 ou 30, **caractérisée en ce que** l'actif est choisi parmi le triclosan, le triclocarban, l'acide salicylique, et leurs mélanges.

32. Composition selon l'une quelconque des revendications 29 à 31, **caractérisée en ce que** l'actif est un filtre solaire choisi parmi les filtres organiques, les filtres physiques et leurs mélanges.

33. Composition selon la revendication 32, **caractérisée en ce que** le filtre physique est choisi parmi les oxydes de titane, les oxydes de zinc et leurs mélanges.

34. Composition selon la revendication 28, **caractérisée en ce que** le tensioactif moussant est choisi parmi les tensioactifs non ioniques, anioniques, amphotères et zwitterioniques, et leurs mélanges.

35. Composition cosmétique et/ou dermatologique sous forme d'émulsion huile-dans-eau comportant une phase huileuse dispersée dans une phase aqueuse, **caractérisée en ce qu**'elle comprend de 0 à environ 1 % en poids de tensioactif émulsionnant, par rapport au poids total de la composition, et qu'elle est conforme à la composition selon l'une quelconque des revendications précédentes.

36. Composition selon l'une quelconque des revendications précédentes, **caractérisée en ce qu**'elle constitue un produit de soin, de traitement, de protection, de nettoyage, de démaquillage et /ou de maquillage des matières kératiniques.

37. Composition selon la revendication précédente, **caractérisée en ce que** la matière kératinique est la peau.

38. Utilisation d'une composition cosmétique selon l'une quelconque des revendications 1 à 35, comme produit de soin pour la peau, les cheveux, le cuir chevelu, des cils, des sourcils, des ongles ou des muqueuses.

39. Utilisation d'une composition cosmétique selon l'une quelconque des revendications 1 à 35, comme produit de maquillage.

40. Utilisation d'une composition cosmétique selon l'une quelconque des revendications 1 à 35, comme produit de protection solaire.

41. Utilisation d'une composition cosmétique selon l'une quelconque des revendications 1 à 35, comme produit capillaire rincé ou non-rincé.

42. Utilisation d'une composition cosmétique selon l'une quelconque des revendications 1 à 35, comme produit de nettoyage et/ou de démaquillage de la peau et/ou des yeux.

43. Procédé de traitement cosmétique d'une matière kératinique, **caractérisé en ce qu**'on applique sur la matière kératinique, une composition cosmétique telle que définie selon l'une quelconque des revendications 1 à 35.

44. Procédé selon la revendication précédente, **caractérisé en ce que** la matière kératinique est la peau.

45. Utilisation d'au moins un polymère hydrosoluble ou hydrodispersible, de structure diblocs A-B où A est un bloc polymérique hydrosoluble ionique et B un bloc polymérique hydrophobe, et où la quantité de bloc polymérique A est égale ou supérieure à 60 % du poids total du polymère diblocs A-B, pour la gélification d'une composition cosmétique et/ou dermatologique comprenant au moins une phase aqueuse et au moins une phase huileuse.

46. Utilisation selon la revendication précédente, **caractérisée en ce que** le bloc polymèrique ionique A est totalement hydrophile et le bloc polymérique B est totalement hydrophobe.

47. Utilisation selon la revendication 45 ou 46, **caractérisée en ce que** la composition comprend en outre un polymère hydrosoluble ou hydrodispersible diblocs A'-B dans lequel A' est un bloc polymérique hydrosoluble neutre et B un bloc polymérique hydrophobe.

## Claims

1. Cosmetic and/or dermatological composition, **characterized in that** it comprises at least one aqueous phase including at least one water-soluble or water-dispersible polymer, of diblock structure A-B in which A is an ionic water-soluble polymer block and B is a hydrophobic polymer block, the amount of polymer block A being greater than or equal to 60% of the total weight of the diblock polymer, and **in that** it comprises at least one oily phase.

2. Composition according to Claim 1, **characterized in that** the amount of polymer block A is greater than or equal to 70% of the total weight of the diblock polymer.

3. Composition according to Claim 1 or 2, **characterized in that** the ionic water-soluble block A is obtained from one or more water-soluble monomers (Ia) or salts thereof, the monomers (Ia) being chosen from:
- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- dimethyldiallylammonium chloride,
- methylvinylimidazolium chloride,
- ethylenic carboxybetaines or sulfobetaines,
- water-soluble vinyl monomers of formula (I) below: 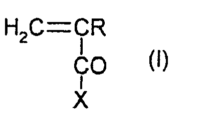
in which:
- R is chosen from H, -CH₃, -C₂H₅ and -C₃H₇;
- X is chosen from:
- alkyl oxides of the type -OR₁ in which R₁ is a linear or branched, saturated or unsaturated hydrocarbon radical containing from 1 to 6 carbon atoms, substituted with at least one carboxylate; sulfonic; sulfate; phosphate and/or quaternary ammonium (-N⁺R₂R₃R₄) group, the radicals R₂, R₃ and R₄ being, independently of each other, a linear or branched, saturated or unsaturated hydrocarbon radical containing 1 to 6 carbon atoms, with the proviso that the sum of the carbon atoms of R₁ + R₂ + R₃ + R₄ does not exceed 6; the radical R₁ optionally being substituted with a halogen atom; a hydroxyl; carboxylic acid; ether; primary amine; secondary amine (-NHR₅); or tertiary amine (-NR₅R₆) group, the radicals R₅ and R₆ being, independently of each other, a linear or branched, saturated or unsaturated hydrocarbon radical containing 1 to 6 carbon atoms, with the proviso that the sum of the carbon atoms of R₁ + R₅ + R₆ does not exceed 6;
- groups -NH₂, -NHR₇ and -NR₇R₈ in which R₇ and R₈ are, independently of each other, a linear or branched, saturated or unsaturated hydrocarbon radical containing 1 to 6 carbon atoms, with the proviso that the total number of carbon atoms of R₇ + R₈ does not exceed 6, the said radicals R₇ and/or R₈ being substituted with at least one carboxylate; sulfonic; sulfate; phosphate; and/or quaternary amine (-N⁺R₉R₁₀R₁₁) group, the radicals R₉, R₁₀ and R₁₁ being, independently of each other, a linear or branched, saturated or unsaturated hydrocarbon radical containing 1 to 6 carbon atoms, with the proviso that the sum of the carbon atoms of R₇ + R₈ + R₉ + R₁₀ + R₁₁ does not exceed 6; the radicals R₇ and/or R₈ optionally being substituted with a halogen atom; or a hydroxyl; carboxylic acid; ether; primary amine; secondary amine (-NHR₅); or tertiary amine (-NR₅R₆) group, in which R₅ and R₆ have the meanings given above, with the proviso that the sum of the carbon atoms of R₇ + R₈ + R₅ + R₆ does not exceed 6;
- the groups R and X being such that the monomer of formula (I) is water-soluble;
- mixtures of these monomers.

4. Composition according to the preceding claim, **characterized in that** the ionic water-soluble block A is also obtained from one or more monomers chosen from hydrophobic monomers (Ib), neutral water-soluble monomers (Ic), and mixtures thereof.

5. Composition according to the preceding claim, **characterized in that** the hydrophobic monomer (Ib) is chosen from:
- styrene and its derivatives;
- vinyl acetate;
- vinyl ethers of formula CH₂=CHOR₁₂ in which R₁₂ is a linear or branched, saturated or unsaturated hydrocarbon radical containing from 1 to 6 carbon atoms;
- acrylonitrile;
- caprolactone;
- vinyl chloride and vinylidene chloride;
- unsaturated silicone monomers,
- hydrophobic vinyl monomers of formula (II) below: 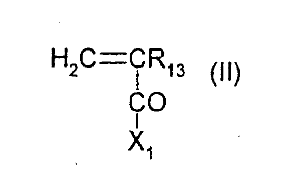
in which:
- R₁₃ is chosen from H, -CH₃, -C₂H₅ and -C₃H₇;
- X₁ is chosen from:
- alkyl oxides of the type -OR₁₄ in which R₁₄ is a linear or branched, saturated or unsaturated hydrocarbon radical containing from 1 to 6 carbon atoms;
- groups -NH₂, -NHR₁₅ and -NR₁₅R₁₆ in which R₁₅ and R₁₆ are, independently of each other, linear or branched, saturated or unsaturated hydrocarbon radicals containing 1 to 6 carbon atoms, with the proviso that the total number of carbon atoms of R₁₅ + R₁₆ does not exceed 6;
- the substituents R₁₃ and X₁ being such that the monomer of formula (II) is hydrophobic;
- mixtures of these monomers.

6. Composition according to Claim 4 or 5, **characterized in that** the neutral monomer (Ic) is chosen from:
- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams including a cyclic alkyl group containing from 4 to 9 carbon atoms,
- vinyl alcohol,
- water-soluble vinyl monomers of formula (III) below: 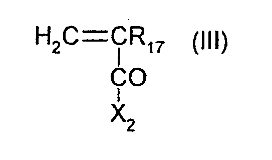
in which:
- R₁₇ is chosen from H, -CH₃, -C₂H₅ and -C₃H₇
- X₂ is chosen from:
- alkyl oxides of the type -OR₁₈ in which R₁₈ is a linear or branched, saturated or unsaturated hydrocarbon radical containing from 1 to 6 carbon atoms, optionally substituted with a halogen atom; a hydroxyl; carboxylic acid; ether; primary amine; secondary amine (-NHR₁₉) or tertiary amine (-NR₁₉R₂₀) group; the radicals R₁₉ and R₂₀ being, independently of each other, a linear or branched, saturated or unsaturated hydrocarbon radical containing 1 to 6 carbon atoms, with the proviso that the sum of the carbon atoms of R₁₈ + R₁₉ + R₂₀ does not exceed 6;
- groups -NH₂, -NHR₂₁ and -NR₂₁R₂₂ in which R₂₁ and R₂₂ are, independently of each other, linear or branched, saturated or unsaturated hydrocarbon radicals containing 1 to 6 carbon atoms, with the proviso that the total number of carbon atoms of R₂₁ + R₂₂ does not exceed 6, the said radicals R₂₁ and R₂₂ optionally being substituted with a halogen atom; a carboxylic acid; hydroxyl; ether; primary amine; secondary amine (-NHR₂₃); or tertiary amine (-NR₂₃R₂₄) group, the radicals R₂₃ and R₂₄ being, independently of each other, a linear or branched, saturated or unsaturated hydrocarbon radical containing 1 to 6 carbon atoms, with the proviso that the sum of the carbon atoms of R₂₁ + R₂₂ + R₂₃ + R₂₄ does not exceed 6;
- the substituents R₁₇ and X₂ being such that the monomer of formula (III) is water-soluble;
- mixtures of these monomers.

7. Composition according to Claim 1, **characterized in that** the ionic water-soluble block A is polyethyleneimine.

8. Composition according to any one of the preceding claims, **characterized in that** the ionic water-soluble block A is totally or partially neutralized with a mineral or organic base.

9. Composition according to any one of the preceding claims, **characterized in that** the hydrophobic block B is obtained from one or more hydrophobic monomers (Id) chosen from:
- styrene and its derivatives,
- vinyl acetate,
- vinyl ethers of formula CH₂=CHOR₂₅ in which R₂₅ is a linear or branched, saturated or unsaturated hydrocarbon radical containing from 1 to 22 carbon atoms,
- acrylonitrile,
- vinyl chloride and vinylidene chloride,
- caprolactone,
- alkenes,
- unsaturated silicone monomers,
- silicone derivatives,
- hydrophobic vinyl monomers of formula (IV) below: 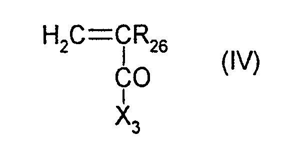
in which:
- R₂₆ is chosen from H, -CH₃, -C₂H₅ or -C₃H₇;
- X₃ is chosen from:
- alkyl oxides of the type -OR₂₇ in which R₂₇ is a linear or branched, saturated or unsaturated hydrocarbon radical containing from 1 to 22 carbon atoms, optionally substituted with a halogen atom; a carboxylate; sulfonic; sulfate; phosphate; hydroxyl; carboxylic acid; ether; primary amine; secondary amine (-NHR₂₈) ; tertiary amine (-NR₂₈R₂₉) or quaternary ammonium (-N⁺R₂₈R₂₉R₃₀) group, the radicals R₂₈, R₂₉ and R₃₀ being, independently of each other, a linear or branched, saturated or unsaturated hydrocarbon radical containing 1 to 22 carbon atoms, with the proviso that the sum of the carbon atoms of R₂₇ + R₂₈ + R₂₉ + R₃₀ does not exceed 22; or R₂₇ is a perfluoroalkyl radical containing from 1 to 18 carbon atoms;
- groups -NH₂, -NHR₃₁ and -NR₃₁R₃₂ in which the radicals R₃₁ and R₃₂ are, independently of each other, a linear or branched, saturated or unsaturated hydrocarbon radical containing 1 to 22 carbon atoms, with the proviso that the total number of carbon atoms of R₃₁ + R₃₂ does not exceed 22, the said radicals R₃₁ and R₃₂ optionally being substituted with a halogen atom; a hydroxyl; ether; carboxylate; sulfonic; sulfate; phosphate; carboxylic acid; primary amine; secondary amine (-NHR₂₈); tertiary amine (-NR₂₈R₂₉) or quaternary ammonium (-N⁺R₂₈R₂₉R₃₀) group, in which R₂₈, R₂₉ and R₃₀ have the same meanings as those given above, with the proviso that the sum of the carbon atoms of R₃₁ + R₃₂ + R₂₈ + R₂₉ + R₃₀ does not exceed 22; or alternatively R₃₁ and R₃₂, independently of each other, are a perfluoroalkyl radical containing from 1 to 18 carbon atoms;
- the substituents R₂₆ and X₃ being such that the monomer of formula (IV) is hydrophobic;
- and mixtures of these monomers.

10. Composition according to the preceding claim, **characterized in that** the hydrophobic block B is also obtained from one or more ionic or neutral water-soluble monomers (Ie) chosen from the following monomers, or salts thereof:
- (meth)acrylic acid;
- styrenesulfonic acid;
- vinylsulfonic acid and (meth)allylsulfonic acid;
- vinylphosphonic acid;
- maleic anhydride;
- maleic acid;
- itaconic acid;
- crotonic acid;
- dimethyldiallylammonium chloride;
- methylvinylimidazolium chloride;
- (meth)acrylamide;
- N-vinylacetamide and N-methyl-N-vinylacetamide;
- N-vinylformamide and N-methyl-N-vinylformamide;
- N-vinyllactams including a cyclic alkyl group containing from 4 to 9 carbon atoms,
- vinyl alcohol;
- 2-vinylpyridine and 4-vinylpyridine;
- the water-soluble vinyl monomers of formula (V) below: 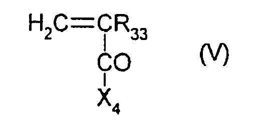
in which:
- R₃₃ is chosen from H, -CH₃, -C₂H₅ and -C₃H₇;
- X₄ is chosen from:
- alkyl oxides of the type -OR₃₄ in which R₃₄ is a linear or branched, saturated or unsaturated hydrocarbon radical containing from 1 to 6 carbon atoms, optionally substituted with a halogen atom; a carboxylate; carboxylic acid; sulfonic; sulfate; phosphate; hydroxyl; ether; primary amine; secondary amine (-NHR₃₅); tertiary amine (-NR₃₅R₃₆); or quaternary ammonium (-N⁺R₃₅R₃₆R₃₇) group, the radicals R₃₅, R₃₆ and R₃₇ being, independently of each other, a linear or branched, saturated or unsaturated hydrocarbon radical containing 1 to 6 carbon atoms, with the proviso that the sum of the carbon atoms of R₃₄ + R₃₅ + R₃₆ + R₃₇ does not exceed 6;
- groups -NH₂, -NHR₃₈ and -NR₃₈R₃₉ in which the radicals R₃₈ and R₃₉ are, independently of each other, linear or branched, saturated or unsaturated hydrocarbon radicals containing 1 to 6 carbon atoms, with the proviso that the total number of carbon atoms of R₃₈ + R₃₉ does not exceed 6, the said radicals R₃₈ and/or R₃₉ optionally being substituted with a halogen atom; a carboxylate; carboxylic acid; sulfonic; sulfate; phosphate; hydroxyl; ether; primary amine; secondary amine (-NHR₃₅); tertiary amine (-NR₃₅R₃₆) or quaternary ammonium (-N⁺R₃₅R₃₆R₃₇) group, in which R₃₅, R₃₆ and R₃₇ have the same meaning as mentioned above, with the proviso that the sum of the carbon atoms of R₃₈ + R₃₉ + R₃₅ + R₃₆ + R₃₇ does not exceed 6;
- the substituents R₃₃ and X₄ being such that the monomer of formula (V) is water-soluble;
- and mixtures of these monomers.

11. Composition according to any one of the preceding claims, **characterized in that** the diblock polymer has a molar mass ranging from 1000 g/mol to 500 000 g/mol.

12. Composition according to any one of the preceding claims, **characterized in that** the diblock polymer has a molar mass ranging from 2000 g/mol to 300 000 g/mol.

13. Composition according to any one of Claims 1 to 3, 7 to 9 and 11 and 12, **characterized in that** the ionic polymer block A is totally water-soluble and the polymer block B is totally hydrophobic.

14. Composition according to the preceding claim, **characterized in that** the diblock polymer comprises sodium polyacrylate as block A and polystyrene as block B.

15. Composition according to any one of the preceding claims, **characterized in that** the amount of diblock polymer(s) ranges from 0.01% to 20% by weight relative to the total weight of the composition.

16. Composition according to any one of the preceding claims, **characterized in that** it also contains at least one neutral diblock water-soluble or water-dispersible polymer A'-B in which A' is a neutral water-soluble polymer block and B is a hydrophobic polymer block in accordance with Claims 9 and 10.

17. Composition according to the preceding claim, **characterized in that** the amount of ionic diblock polymer A-B is greater than 10% by weight relative to the total amount of polymers A-B and A'-B.

18. Composition according to Claim 16 or 17, **characterized in that** the block A' is obtained from one or more water-soluble monomers chosen from the monomers (If) below:
- (meth)acrylamide,
- N-vinylacetamide and N-methyl-N-vinylacetamide,
- N-vinylformamide and N-methyl-N-vinylformamide,
- N-vinyllactams including a cyclic alkyl group containing from 4 to 9 carbon atoms,
- vinyl alcohol,
- maleic anhydride,
- vinylamine,
- water-soluble vinyl monomers of formula (VI) below: 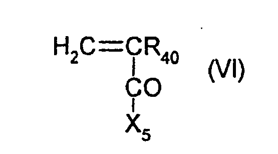
in which:
- R₄₀ is chosen from H, -CH₃, -C₂H₅ and -C₃H₇;
- X₅ is chosen from:
- alkyl oxides of the type -OR₄₁ in which R₄₁ is a linear or branched, saturated or unsaturated hydrocarbon radical containing from 1 to 6 carbon atoms, optionally substituted with a halogen atom; a carboxylic acid; hydroxyl; ether; primary amine; secondary amine (-NHR₄₂); or tertiary amine (-NR₄₂R₄₃) group; the radicals R₄₂ and R₄₃ being, independently of each other, a linear or branched, saturated or unsaturated hydrocarbon radical containing 1 to 6 carbon atoms, with the proviso that the sum of the carbon atoms of R₄₁ + R₄₂ + R₄₃ does not exceed 6;
- groups -NH₂, -NHR₄₄ and -NR₄₄R₄₅ in which R₄₄ and R₄₅ are, independently of each other, linear or branched, saturated or unsaturated hydrocarbon radicals containing 1 to 6 carbon atoms, with the proviso that the total number of carbon atoms of R₄₄ + R₄₅ does not exceed 6, the said radicals R₄₄ and R₄₅ optionally being substituted with a halogen atom; a carboxylic acid; hydroxyl; ether; primary amine; secondary amine (-NHR₄₆); or tertiary amine (-NR₄₆R₄₇) group, the radicals R₄₆ and R₄₇ being, independently of each other, a linear or branched, saturated or unsaturated hydrocarbon radical containing 1 to 6 carbon atoms, with the proviso that the sum of the carbon atoms of R₄₄ + R₄₅ + R₄₆ and R₄₇ does not exceed 6;
- the substituents R₄₀ and X₅ being such that the monomer of formula (VI) is water-soluble;
- and mixtures of these monomers.

19. Composition according to the preceding claim, **characterized in that** the neutral water-soluble block A' is also obtained from one or more hydrophobic monomers (Ig) chosen from the following monomers:
- styrene and its derivatives,
- vinyl acetate,
- vinyl ethers of formula CH₂=CHOR₄₈ in which R₄₈ is a linear or branched, saturated or unsaturated hydrocarbon radical containing from 1 to 6 carbon atoms;
- acrylonitrile,
- caprolactone,
- vinyl chloride and vinylidene chloride,
- unsaturated silicone monomers,
- the hydrophobic vinyl monomers of formula (VII) below: 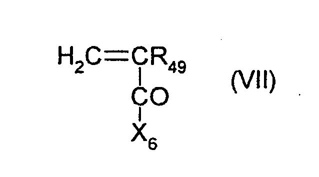
in which:
- R₄₉ is chosen from H, -CH₃, -C₂H₅ and -C₃H₇;
- X₆ is chosen from:
- alkyl oxides of the type -OR₅₀ in which R₅₀ is a linear or branched, saturated or unsaturated hydrocarbon radical containing from 1 to 6 carbon atoms;
- groups -NH₂, -NHR₅₁ and -NR₅₁R₅₂ in which R₅₁ and R₅₂ are, independently of each other, linear or branched, saturated or unsaturated hydrocarbon radicals containing 1 to 6 carbon atoms, with the proviso that the total number of carbon atoms of R₅₁ + R₅₂ does not exceed 6;
- the substituents R₄₉ and X₆ being such that the monomer of formula (VII) is hydrophobic;
- and mixtures of these monomers.

20. Composition according to any one of Claims 16 to 19, **characterized in that** the neutral diblock polymer has a molar mass ranging from 1000 g/mol to 500 000 g/mol.

21. Composition according to any one of Claims 16 to 20, **characterized in that** the amount of neutral hydrophilic block A' in the diblock polymer A'-B is greater than 50% of the total weight of the diblock polymer.

22. Composition according to any one of the preceding claims, **characterized in that** it contains a physiologically acceptable medium.

23. Composition according to any one of the preceding claims, **characterized in that** it also contains at least one organic solvent chosen from the group consisting of hydrophilic organic solvents, lipophilic organic solvents and amphiphilic solvents, and mixtures thereof.

24. Composition according to the preceding claim, **characterized in that** the organic solvents are chosen from the group consisting of monoalcohols containing from 1 to 8 carbon atoms, polyols, monoalkyl and dialkyl isosorbides, polyethylene glycols, ethylene glycol ethers, propylene glycol ethers, polyol ethers, polyol esters, fatty acid alkyl esters, and mixtures thereof.

25. Composition according to Claim 23 or 24, **characterized in that** the organic solvent(s) represent (s) from 5% to 50% of the total weight of the composition.

26. Composition according to any one of the preceding claims, **characterized in that** the oily phase comprises at least one oil.

27. Composition according to any one of the preceding claims, **characterized in that** it constitutes an O/W, W/O, W/O/W or O/W/O emulsion.

28. Composition according to any one of the preceding claims, **characterized in that** it also contains at least one adjuvant chosen from the group consisting of gelling agents and/or thickeners; polymers; foaming surfactants; moisturizers; emollients; hydrophilic or lipophilic active agents; free-radical scavengers; sequestering agents; antioxidants; preserving agents; acidifying or basifying agents; fragrances; pigments; fillers; film-forming agents; dyestuffs; and mixtures thereof.

29. Composition according to the preceding claim, **characterized in that** the active agent is chosen from protein hydrolysates; anti-inflammatory agents; polyols; sugar derivatives; natural extracts; procyannidol oligomers; vitamins; urea; caffeine; depigmenting agents; salicylic acid and its derivatives; α-hydroxy acids; retinoids; sunscreens; hydrocortisone; melatonin; algal, fungal, plant, yeast or bacterial extracts; enzymes; DHEA and its derivatives and metabolites; antibacterial active agents; matt-effect agents; tensioning agents; and mixtures thereof.

30. Composition according to the preceding claim, **characterized in that** the active agent is chosen from vitamin A, vitamin C, vitamin E, vitamin B5, vitamin B3, esters thereof and mixtures thereof.

31. Composition according to Claim 29 or 30, **characterized in that** the active agent is chosen from Triclosan, Triclocarban, salicylic acid, and mixtures thereof.

32. Composition according to any one of Claims 29 to 31, **characterized in that** the active agent is a sunscreen chosen from organic screening agents and physical sunblock screening agents, and mixtures thereof.

33. Composition according to Claim 32, **characterized in that** the physical sunblock screening agent is chosen from titanium oxides and zinc oxides, and mixtures thereof.

34. Composition according to Claim 28, **characterized in that** the foaming surfactant is chosen from nonionic, anionic, amphoteric and zwitterionic surfactants, and mixtures thereof.

35. Cosmetic and/or dermatological composition in the form of an oil-in-water emulsion including an oily phase dispersed in an aqueous phase, **characterized in that** it comprises from 0 to about 1% by weight of emulsifying surfactant relative to the total weight of the composition, and **in that** it is in accordance with the composition according to any one of the preceding claims.

36. Composition according to any one of the preceding claims, **characterized in that** it constitutes a care, treatment, protective, cleansing, makeup-removing and/or makeup product for keratin materials.

37. Composition according to the preceding claim, **characterized in that** the keratin material is the skin.

38. Use of a cosmetic composition according to any one of Claims 1 to 35, as a care product for the skin, the hair, the scalp, the eyelashes, the eyebrows, the nails or mucous membranes.

39. Use of a cosmetic composition according to any one of Claims 1 to 35, as a makeup product.

40. Use of a cosmetic composition according to any one of Claims 1 to 35, as an antisun product.

41. Use of a cosmetic composition according to any one of Claims 1 to 35, as a rinse-out or leave-in hair product.

42. Use of a cosmetic composition according to any one of Claims 1 to 35, as a product for cleansing and/or removing makeup from the skin and/or the eyes.

43. Cosmetic process for treating a keratin material, **characterized in that** a cosmetic composition as defined according to any one of Claims 1 to 35 is applied to the keratin material.

44. Process according to the preceding claim, **characterized in that** the keratin material is the skin.

45. Use of at least one water-soluble or water-dispersible polymer of diblock structure A-B in which A is an ionic water-soluble polymer block and B is a hydrophobic polymer block, and in which the amount of polymer block A is greater than or equal to 60% of the total weight of the diblock polymer A-B, for gelling a cosmetic and/or dermatological composition comprising at least one aqueous phase and at least one oily phase.

46. Use according to the preceding claim, **characterized in that** the ionic polymer block A is totally hydrophilic and the polymer block B is totally hydrophobic.

47. Use according to Claim 45 or 46, **characterized in that** the composition also comprises a water-soluble or water-dispersible diblock polymer A'-B in which A' is a neutral water-soluble polymer block and B is a hydrophobic polymer block.

## Patentansprüche

1. Kosmetische und/oder dermatologische Zusammensetzung, **dadurch gekennzeichnet, dass** sie mindestens eine wässrige Phase aufweist, die mindestens ein wasserlösliches oder in Wasser dispergierbares Polymer mit einer Zweiblockstruktur A-B enthält, worin A ein ionischer wasserlöslicher Polymerblock ist und B einen hydrophoben Polymerblock bedeutet, wobei der Mengenanteil des Polymerblocks A 60 % des Gesamtgewichts des Zweiblockpolymers ausmacht oder darüber liegt, und **dadurch**, dass sie mindestens eine Ölphase aufweist.

2. Zusammensetzung nach Anspruch 1, **dadurch gekennzeichnet, dass** der Mengenanteil des Polymerblocks A mindestens 70 % des Gesamtgewichts des Zweiblockpolymers beträgt.

3. Zusammensetzung nach Anspruch 1 oder 2, **dadurch gekennzeichnet, dass** der ionische wasserlösliche Block A ausgehend von einem oder mehreren wasserlöslichen Monomeren (Ia) oder deren Salzen erhalten wird, wobei die Monomere (Ia) ausgewählt sind unter:
- (Meth)acrylsäure,
- Styrolsulfonsäure,
- Vinylsulfonsäure und (Meth)allylsulfonsäure,
- Vinylphosphonsäure,
- Maleinsäure,
- Itaconsäure,
- Crotonsäure,
- Dimethyldiallylammoniumchlorid,
- Methylvinylimidazoliumchlorid,
- ethylenischen Carboxybetainen oder Sulfobetainen,
- wasserlöslichen Vinylmonomeren der folgenden Formel (I): 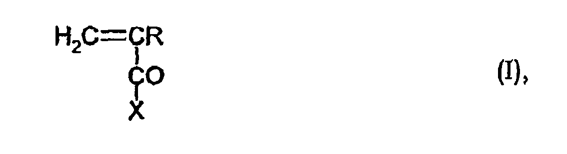
wobei in der Formel:
- R unter H, -CH₃, -C₂H₅ oder -C₃H₇ ausgewählt ist;
- X ausgewählt ist unter:
- Alkoxygruppen vom Typ -OR₁, worin R₁ eine geradkettige oder verzweigte, gesättigte oder ungesättigte Kohlenwasserstoffgruppe mit 1 bis 6 Kohlenstoffatomen bedeutet, die mit mindestens einer Carboxylatgruppe; Sulfonsäuregruppe; Sulfatgruppe; Phosphatgruppe und/oder einer quartären Ammoniumgruppe (-N⁺R₂R₃R₄) substituiert ist, wobei die Gruppen R₂, R₃ und R₄ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₁ + R₂ + R₃ + R₄ 6 nicht übersteigt; wobei die Gruppe R₁ gegebenenfalls substituiert ist mit einem Halogenatom; einer Hydroxygruppe; einer Carboxygruppe; einer Ethergruppe; einer primären Aminogruppe; einer sekundären Aminogruppe (-NHR₅); oder einer tertiären Aminogruppe (-NR₅R₆), wobei die Gruppen R₅ und R₆ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₁ + R₅ + R₆ 6 nicht übersteigt;
- den Gruppen -NH₂, -NHR₇ und -NR₇R₈, wobei R₇ und R₈ unabhängig voneinander eine geradkettige oder verzweigte gesättigte oder ungesättigte Kohlenwasserstoffgruppe mit 1 bis 6 Kohlenstoffatomen bedeuten, mit der Maßgabe, dass die Gesamtzahl der Kohlenstoffatome von R₇ + R₈ 6 nicht übersteigt, wobei die Gruppe R₇ und/oder R₈ mit mindestens einer Carboxylatgruppe; Sulfonsäuregruppe; Sulfatgruppe; Phosphatgruppe und/oder einem quartären Amin (-N⁺R₉R₁₀R₁₁) substituiert ist, wobei die Gruppen R₉, R₁₀ und R₁₁ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₇ + R₈ + R₉ + R₁₀ + R₁₁ 6 nicht übersteigt; wobei die Gruppen R₇ und/oder R₈ gegebenenfalls mit einem Halogenatom; oder einer Hydroxygruppe; einer Carboxygruppe; einer Ethergruppe; einer primären Aminogruppe; einer sekundären Aminogruppe (-NHR₅); oder einer tertiären Aminogruppe (-NR₅R₆) substituiert sind, wobei R₅ und R₆ die oben angegebenen Bedeutungen aufweisen, mit der Maßgabe, dass die Summe der Kohlenstoffatome von R₇ + R₈ + R₅ + R₆ 6 nicht übersteigt;
- wobei die Gruppen R und X so gewählt sind, dass das Monomer der Formel (I)wasserlöslich ist;
- Gemischen dieser Monomere.

4. Zusammensetzung nach dem vorhergehenden Anspruch, **dadurch gekennzeichnet, dass** der ionische wasserlösliche Block A ferner ausgehend von einem oder mehreren Monomeren erhalten wird, die unter den hydrophoben Monomeren (Ib), neutralen wasserlöslichen Monomeren (Ic) und deren Gemischen ausgewählt sind.

5. Zusammensetzung nach dem vorhergehenden Anspruch, **dadurch gekennzeichnet, dass** das hydrophobe Monomer (Ib) ausgewählt ist unter:
- Styrol und seinen Derivaten;
- Vinylacetat;
- Vinylethern der Formel CH₂=CHOR₁₂, worin R₁₂ eine geradkettige oder verzweigte, gesättigte oder ungesättigte Kohlenwasserstoffgruppe mit 1 bis 6 Kohlenstoffatomen ist;
- Acrylnitril;
- Caprolacton,
- Vinylchlorid und Vinylidenchlorid;
- ungesättigten Siliconmonomeren;
- hydrophoben Vinylmonomeren der Formel (II): 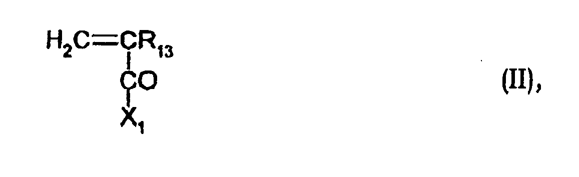
wobei in der Formel:
- R₁₃ unter H, -CH₃, -C₂H₅ oder -C₃H₇ ausgewählt ist;
- X₁ ausgewählt ist unter:
- Alkoxygruppen vom Typ -OR₁₄, wobei R₁₄ eine geradkettige oder verzweigte, gesättigte oder ungesättigte Kohlenwasserstoffgruppe mit 1 bis 6 Kohlenstoffatomen bedeutet;
- den Gruppen -NH₂, -NHR₁₅ und -NR₁₅R₁₆, wobei R₁₅ und R₁₆ unabhängig voneinander geradkettige oder verzweigte, gesättigte oder ungesättigte Kohlenwasserstoffgruppen mit 1 bis 6 Kohlenstoffatomen sind, mit der Maßgabe, dass die Gesamtzahl der Kohlenstoffatome von R₁₅ + R₁₆ 6 nicht übersteigt;
- wobei die Substituenten R₁₃ und X₁ so gewählt sind, dass das Monomer der Formel (II) hydrophob ist;
- Gemischen dieser Monomere.

6. Zusammensetzung nach Anspruch 4 oder 5, **dadurch gekennzeichnet, dass** das neutrale Monomer (Ic) ausgewählt ist unter:
- (Meth)acrylamid;
- N-Vinylacetamid und N-Methyl-N-vinylacetamid;
- N-Vinylformamid und N-Methyl-N-vinylformamid;
- Maleinsäureanhydrid;
- Vinylamin;
- N-Vinyllactamen, die eine cyclische Alkylgruppe mit 4 bis 9 Kohlenstoffatomen aufweisen;
- Vinylalkohol;
- wasserlöslichen Vinylmonomeren der folgenden Formel (III): 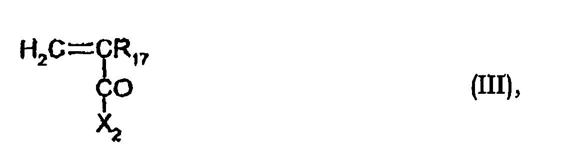
wobei in der Formel:
- R₁₇ unter H, -CH₃, -C₂H₅ oder -C₃H₇ ausgewählt ist;
- X₂ ausgewählt ist unter:
- Alkoxygruppen vom Typ -OR₁₈, wobei R₁₈ eine geradkettige oder verzweigte, gesättigte oder ungesättigte Kohlenwasserstoffgruppe mit 1 bis 6 Kohlenstoffatomen bedeutet, die gegebenenfalls mit einem Halogenatom; einer Hydroxygruppe; einer Carboxygruppe; einer Ethergruppe; einer primären Aminogruppe; einer sekundären Aminogruppe (-NHR₁₉) oder einer tertiären Aminogruppe (-NR₁₉R₂₀) substituiert ist; wobei die Gruppen R₁₉ und R₂₀ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₁₈ + R₁₉ + R₂₀ 6 nicht übersteigt;
- den Gruppen -NH₂, -NHR₂₁ und -NR₂₁R₂₂, wobei R₂₁ und R₂₂ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Gesamtzahl der Kohlenstoffatome von R₂₁ + R₂₂ 6 nicht übersteigt, wobei die Gruppen R₂₁ und R₂₂ gegebenenfalls mit einem Halogenatom; einer Carboxygruppe; einer Hydroxygruppe; einer Ethergruppe; einer primären Aminogruppe; einer sekundären Aminogruppe (-NHR₂₃), einer tertiären Aminogruppe (-NR₂₃R₂₄) substituiert sind, wobei die Gruppen R₂₃ und R₂₄ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₂₁ + R₂₂ + R₂₃ + R₂₄ 6 nicht übersteigt;
- wobei die Substituenten R₁₇ und X₂ so gewählt sind, dass die Monomere der Formel (III) wasserlöslich sind;
- Gemischen dieser Monomere.

7. Zusammensetzung nach Anspruch 1, **dadurch gekennzeichnet, dass** der ionische wasserlösliche Block A das Polyethylenimin ist.

8. Zusammensetzung nach einem der vorhergehenden Ansprüche, **dadurch gekennzeichnet, dass** der ionische wasserlösliche Block A ganz oder teilweise mit einer anorganischen oder organischen Base neutralisiert ist.

9. Zusammensetzung nach einem der vorhergehenden Ansprüche, **dadurch gekennzeichnet, dass** der hydrophobe Block B ausgehend von einem oder mehreren hydrophoben Monomeren (Id) erhalten wird, die ausgewählt sind unter:
- Styrol und seinen Derivaten;
- Vinylacetat;
- Vinylethern der Formel CH₂=CHOR₂₅, wobei R₂₅ eine geradkettige oder verzweigte, gesättigte oder ungesättigte Kohlenwasserstoffgruppe mit 1 bis 22 Kohlenstoffatomen ist,
- Acrylnitril;
- Vinylchlorid und Vinylidenchlorid;
- Caprolacton;
- Alkenen;
- ungesättigten Siliconmonomeren;
- Siliconderivaten;
- hydrophoben Vinylmonomeren der folgenden Formel (IV): 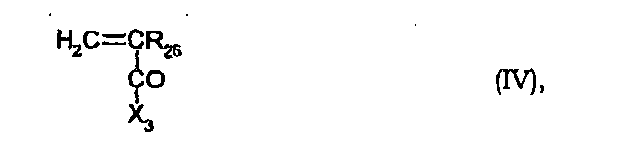
wobei in der Formel:
- R₂₆ unter H, -CH₃, -C₂H₅ oder -C₃H₇ ausgewählt ist;
- X₃ ausgewählt ist unter:
- Alkoxygruppen vom Typ -OR₂₇, wobei R₂₇ eine geradkettige oder verzweigte, gesättigte oder ungesättigte Kohlenwasserstoffgruppe mit 1 bis 22 Kohlenstoffatomen ist, die gegebenenfalls mit einem Halogenatom; einer Carboxylatgruppe; einer Sulfonsäuregruppe; einer Sulfatgruppe; einer Phosphatgruppe; einer Hydroxygruppe; einer Carbonsäuregruppe; einer Ethergruppe; einer primären Aminogruppe; einer sekundären Aminogruppe (-NHR₂₈); einer tertiären Aminogruppe (-NR₂₈R₂₉) oder einer quartären Ammoniumgruppe (-N⁺R₂₈R₂₉R₃₀) substituiert ist, wobei die Gruppen R₂₈, R₂₉ und R₃₀ unabhängig voneinander eine geradkettige oder verzweigte, gesättigte oder ungesättigte Kohlenwasserstoffgruppe mit 1 bis 22 Kohlenstoffatomen bedeuten, mit der Maßgabe, dass die Summe der Kohlenstoffatome von R₂₇ + R₂₈ + R₂₉ + R₃₀ 22 nicht übersteigt; oder R₂₇ ist eine Perfluoralkylgruppe mit 1 bis 18 Kohlenstoffatomen;
- den Gruppen -NH₂, -NHR₃₁ und -NR₃₁R₃₂, worin die Gruppen R₃₁ und R₃₂ unabhängig voneinander eine geradkettige oder verzweigte, gesättigte oder ungesättigte Kohlenwasserstoffgruppe mit 1 bis 22 Kohlenstoffatomen bedeuten, mit der Maßgabe, dass die Gesamtzahl der Kohlenstoffatome von R₃₁ und R₃₂ 22 nicht übersteigt, wobei die Gruppen R₃₁ und R₃₂ gegebenenfalls mit einem Halogenatom; einer Hydroxygruppe; einer Ethergruppe; einer Carboxylatgruppe; einer Sulfonsäuregruppe, einer Sulfatgruppe; einer Phosphatgruppe, einer Carboxygruppe; einer primären Aminogruppe, einer sekundären Aminogruppe (-NHR₂₈); einer tertiären Aminogruppe (-NR₂₈R₂₉) oder einer quartären Ammoniumgruppe (-N⁺R₂₈R₂₉R₃₀) substituiert sind, worin R₂₈, R₂₉ und R₃₀ die oben angegebenen Bedeutungen aufweisen, mit der Maßgabe, dass die Summe der Kohlenstoffatome von R₃₁ + R₃₂ + R₂₈ + R₂₉ + R₃₀ 22 nicht übersteigt; oder R₃₁ und R₃₂ bedeuten unabhängig voneinander eine Perfluoralkylgruppe mit 1 bis 18 Kohlenstoffatomen;
- wobei die Substituenten R₂₆ und X₃ so sind, dass das Monomer der Formel (IV) hydrophob ist;
- und Gemischen dieser Monomere.

10. Zusammensetzung nach dem vorhergehenden Anspruch, **dadurch gekennzeichnet, dass** der hydrophobe Block B ferner ausgehend von einem oder mehreren ionischen oder neutralen wasserlöslichen Monomeren (Ie) erhalten wird, die unter den folgenden Monomeren oder deren Salzen ausgewählt sind:
- (Meth)acrylsäure;
- Styrolsulfonsäure;
- Vinylsulfonsäure und (Meth)allylsulfonsäure;
- Vinylphosphonsäure;
- Maleinsäureanhydrid;
- Maleinsäure;
- Itaconsäure;
- Crotonsäure;
- Dimethyldiallylammoniumchlorid;
- Methylvinylimidazoliumchlorid;
- (Meth)acrylamid;
- N-Vinylacetamid und N-Methyl-N-vinylacetamid;
- N-Vinylformamid und N-Methyl-N-vinylformamid;
- N-Vinyllactamen, die eine cyclische Alkylgruppe mit 4 bis 9 Kohlenstoffatomen enthalten;
- Vinylalkohol;
- 2-Vinylpyridin und 4-Vinylpyridin;
- wasserlöslichen Vinylmonomeren der folgenden Formel (V): 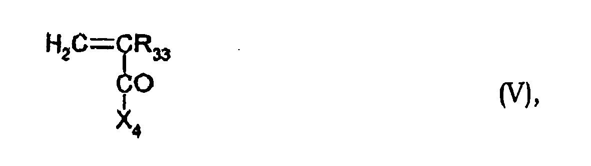
wobei in der Formel:
- R₃₃ unter H, -CH₃, -C₂H₅ oder -C₃H₇ ausgewählt ist;
- X₄ ausgewählt ist unter:
- Alkoxygruppen vom Typ -OR₃₄, worin R₃₄ eine geradkettige oder verzweigte, gesättigte oder ungesättigte Kohlenwasserstoffgruppe mit 1 bis 6 Kohlenstoffatomen ist, die gegebenenfalls mit einem Halogenatom; einer Carboxylatgruppe; einer Carboxygruppe; einer Sulfonsäuregruppe; einer Sulfatgruppe; einer Phosphatgruppe; einer Hydroxygruppe; einer Ethergruppe; einer primären Aminogruppe; einer sekundären Aminogruppe (-NHR₃₅); einer tertiären Aminogruppe (-NR₃₅R₃₆); oder einer quartären Ammoniumgruppe (-N⁺R₃₅R₃₆R₃₇) substituiert ist, wobei die Gruppen R₃₅, R₃₆ und R₃₇ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₃₄ + R₃₅ + R₃₆ + R₃₇ 6 nicht übersteigt;
- den Gruppen -NH₂, -NHR₃₈ und -NR₃₈R₃₉, wobei die Gruppen R₃₈ und R₃₉ unabhängig voneinander geradkettige oder verzweigte, gesättigte oder ungesättigte Kohlenwasserstoffgruppen mit 1 bis 6 Kohlenstoffatomen sind, mit der Maßgabe, dass die Gesamtzahl der Kohlenstoffatome von R₃₈ + R₃₉ 6 nicht übersteigt, wobei die Gruppe R₃₈ und/oder R₃₉ gegebenenfalls mit einem Halogenatom; mit einer Carboxylatgruppe; einer Carbonsäuregruppe; einer Sulfonsäuregruppe; einer Sulfatgruppe; einer Phosphatgruppe; einer Hydroxygruppe; einer Ethergruppe; einer primären Aminogruppe; einer sekundären Aminogruppe (-NHR₃₅); einer tertiären Aminogruppe (-NR₃₅R₃₆) oder einer quartären Ammoniumgruppe (-N⁺R₃₅R₃₆R₃₇) substituiert ist, wobei R₃₅, R₃₆ und R₃₇ die oben angegebenen Bedeutungen aufweisen, mit der Maßgabe, dass die Summe der Kohlenstoffatome von R₃₈ + R₃₉ + R₃₅ + R₃₆ + R₃₇ 6 nicht übersteigt;
- wobei die Substituenten R₃₃ und X₄ so gewählt sind, dass das Monomer der Formel (V) wasserlöslich ist;
- und Gemischen dieser Monomere.

11. Zusammensetzung nach einem der vorhergehenden Ansprüche, **dadurch gekennzeichnet, dass** das Zweiblockpolymer eine Molmasse von 1 000 bis 500 000 g/mol besitzt.

12. Zusammensetzung nach einem der vorhergehenden Ansprüche, **dadurch gekennzeichnet, dass** das Zweiblockpolymere eine Molmasse von 2 000 bis 300 000 g/mol aufweist.

13. Zusammensetzung nach einem der Ansprüche 1 bis 3, 7 bis 9 und 11 bis 12, **dadurch gekennzeichnet, dass** der ionische Polymerblock A vollständig wasserlöslich und der Polymerblock B vollständig hydrophob ist.

14. Zusammensetzung nach einem der vorhergehenden Ansprüche, **dadurch gekennzeichnet, dass** das Zweiblockpolymer als Block A Natriumpolyacrylat und als Block B Polystyrol enthält.

15. Zusammensetzung nach einem der vorhergehenden Ansprüche, **dadurch gekennzeichnet, dass** der Mengenanteil des Zweiblockpolymers oder der Zweiblockpolymere im Bereich von 0,01 bis 20 Gew.-%, bezogen auf das Gesamtgewicht der Zusammensetzung, liegt.

16. Zusammensetzung nach einem der vorhergehenden Ansprüche, **dadurch gekennzeichnet, dass** sie ferner mindestens ein wasserlösliches oder in Wasser dispergierbares neutrales Zweiblockpolymer A'-B enthält, worin A' einen neutralen wasserlöslichen Polymerblock und B einen hydrophoben Polymerblock nach einem der Ansprüche 9 und 10 bedeutet.

17. Zusammensetzung nach dem vorhergehenden Anspruch, **dadurch gekennzeichnet, dass** der Mengenanteil des ionischen Zweiblockpolymers A-B, bezogen auf die Gesamtmenge der Polymere A-B und A'-B, größer als 10 Gew.-% ist.

18. Zusammensetzung nach einem der Ansprüche 16 oder 17, **dadurch gekennzeichnet, dass** der Block A' aus einem oder mehreren wasserlöslichen Monomeren erhalten wird, die unter den folgenden Monomeren (If) ausgewählt sind:
- (Meth)acrylamid;
- N-Vinylacetamid und N-Methyl-N-vinylacetamid;
- N-Vinylformamid und N-Methyl-N-vinylformamid;
- N-Vinyllactamen, die eine cyclische Alkylgruppe mit 4 bis 9 Kohlenstoffatomen enthalten;
- Vinylalkohol,
- Maleinsäureanhydrid;
- Vinylamin;
- wasserlöslichen Vinylmonomeren der folgenden Formel (VI): 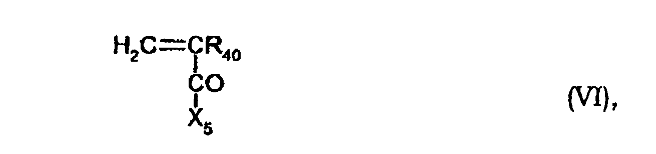
wobei in der Formel:
- R₄₀ unter H, -CH₃, -C₂H₅ oder -C₃H₇ ausgewählt ist;
- X₅ ausgewählt ist unter:
- Alkoxygruppen vom Typ -OR₄₁, worin R₄₁ eine geradkettige oder verzweigte, gesättigte oder ungesättigte Kohlenwasserstoffgruppe mit 1 bis 6 Kohlenstoffatomen bedeutet, die gegebenenfalls substituiert ist mit einem Halogenatom; einer Carbonsäuregruppe; einer Hydroxygruppe; einer Ethergruppe; einer primären Aminogruppe; einer sekundären Aminogruppe (-NHR₄₂) oder einer tertiären Aminogruppe (-NR₄₂R₄₃), wobei die Gruppen R₄₂ und R₄₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₄₁ + R₄₂ + R₄₃ 6 nicht übersteigt;
- den Gruppen -NH₂, -NHR₄₄ und -NR₄₄R₄₅, worin R₄₄ und R₄₅ unabhängig voneinander geradkettige oder verzweigte, gesättigte oder ungesättigte Kohlenwasserstoffgruppen mit 1 bis 6 Kohlenstoffatomen bedeuten, mit der Maßgabe, dass die Gesamtzahl der Kohlenstoffatome von R₄₄ + R₄₅ 6 nicht übersteigt, wobei die Gruppen R₄₄ und R₄₅ gegebenenfalls substituiert sind mit einem Halogenatom; einer Carboxygruppe; einer Hydroxygruppe; einer Ethergruppe; einer primären Aminogruppe; einer sekundären Aminogruppe (-NHR₄₆); oder einer tertiären Aminogruppe (-NR₄₆R₄₇), wobei die Gruppen R₄₆ und R₄₇ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₄₄+ R₄₅ + R₄₆ + R₄₇ 6 nicht übersteigt;
- wobei die Substituenten R₄₀ und X₅ so sind, dass das Monomer der Formel (VI) wasserlöslich ist;
- und Gemischen dieser Monomere.

19. Zusammensetzung nach dem vorhergehenden Anspruch, **dadurch gekennzeichnet, dass** der neutrale wasserlösliche Block A' ferner ausgehend von einem oder mehreren hydrophoben Monomeren (Ig) erhalten wird, die unter den folgenden Monomeren ausgewählt sind:
- Styrol und seinen Derivaten;
- Vinylacetat;
- Vinylethern der Formel CH₂=CHOR₄₈, worin R₄₈ eine geradkettige oder verzweigte, gesättigte oder ungesättigte Kohlenwasserstoffgruppe mit 1 bis 6 Kohlenstoffatomen ist;
- Acrylnitril;
- Caprolacton;
- Vinylchlorid und Vinylidenchlorid;
- ungesättigten Siliconmonomeren;
- hydrophoben Vinylmonomeren der folgenden Formel (VII): 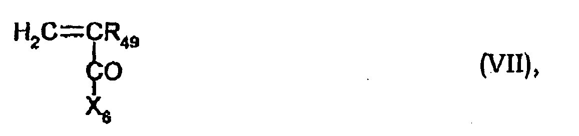
wobei in der Formel:
- R₄₉ unter H, -CH₃, -C₂H₅ oder -C₃H₇ ausgewählt ist;
- X₆ ausgewählt ist unter:
- Alkoxygruppen vom Typ -OR₅₀, worin R₅₀ eine geradkettige oder verzweigte, gesättigte oder ungesättigte Kohlenwasserstoffgruppe mit 1 bis 6 Kohlenstoffatomen bedeutet;
- den Gruppen -NH₂, -NHR₅₁ und -NR₅₁R₅₂, worin R₅₁ und R₅₂ unabhängig voneinander geradkettige oder verzweige, gesättigte oder ungesättigte Kohlenwasserstoffgruppen mit 1 bis 6 Kohlenstoffatomen bedeuten, mit der Maßgabe, dass die Gesamtzahl der Kohlenstoffatome von R₅₁ und R₅₂ 6 nicht übersteigt;
- wobei die Substituenten R₄₉ und X₆ so gewählt sind, dass das Monomer der Formel (VII) hydrophob ist;
- und Gemischen dieser Monomere.

20. Zusammensetzung nach einem der Ansprüche 16 bis 19, **dadurch gekennzeichnet, dass** das neutrale Zweiblockpolymere eine Molmasse von 1 000 bis 500 000 g/mol aufweist.

21. Zusammensetzung nach einem der Ansprüche 16 bis 20, **dadurch gekennzeichnet, dass** der Mengenanteil des neutralen hydrophilen Blocks A' in dem Zweiblockpolymer A'-B über 50 % des Gesamtgewichts des Zweiblockpolymers liegt.

22. Zusammensetzung nach einem der vorhergehenden Ansprüche, **dadurch gekennzeichnet, dass** sie ein physiologisch akzeptables Medium enthält.

23. Zusammensetzung nach einem der vorhergehenden Ansprüche, **dadurch gekennzeichnet, dass** sie ferner mindestens ein organisches Lösungsmittel enthält, das unter den hydrophilen organischen Lösungsmitteln, lipophilen organischen Lösungsmitteln, amphiphilen Lösungsmitteln oder deren Gemischen ausgewählt ist.

24. Zusammensetzung nach dem vorhergehenden Anspruch, **dadurch gekennzeichnet, dass** die organischen Lösungsmittel unter den Monoalkoholen mit 1 bis 8 Kohlenstoffatomen, Polyolen, Mono- und Dialkylisosorbiden, Polyethylenglycolen, Ethylenglycolethern, Propylenglycolethern, Polyolethern, Polyolestern, Fettsäurealkylestern und deren Gemischen ausgewählt sind.

25. Zusammensetzung nach Anspruch 23 oder 24, **dadurch gekennzeichnet, dass** das oder die organischen Lösungsmittel 5 bis 50 % des Gesamtgewichts der Zusammensetzung ausmachen.

26. Zusammensetzung nach einem der vorhergehenden Ansprüche, **dadurch gekennzeichnet, dass** die Ölphase mindestens ein Öl enthält.

27. Zusammensetzung nach einem der vorhergehenden Ansprüche, **dadurch gekennzeichnet, dass** sie eine Emulsion O/W, W/O, W/O/W, O/W/O ist.

28. Zusammensetzung nach einem der vorhergehenden Ansprüche, **dadurch gekennzeichnet, dass** sie ferner mindestens einen Zusatzstoff enthält, der ausgewählt ist unter Gelbildnern und/oder Verdickungsmittel; Polymeren; schäumenden grenzflächenaktiven Stoffen; Hydratisierungsmitteln; Emollientien; hydrophilen oder lipophilen Wirkstoffen; Radikalfängern für freie Radikale; Maskierungsmitteln; Antioxidantien; Konservierungsmitteln; Alkalisierungsmitteln oder Ansäuerungsmitteln; Parfums; Pigmenten; Füllstoffen; Filmbildnern; Farbmitteln; und deren Gemischen.

29. Zusammensetzung nach dem vorhergehenden Anspruch, **dadurch gekennzeichnet, dass** der Wirkstoff unter den Proteinhydrolysaten; entzündungshemmenden Wirkstoffen; Polyolen; Zuckerderivaten; natürlichen Extrakten; Procyanidin-Oligomeren; Vitaminen; Harnstoff; Coffein; depigmentierenden Wirkstoffen; Salicylsäure und ihren Derivaten; α-Hydroxysäuren; Retinoiden; Sonnenschutzfiltern; Hydrocortison; Melatonin; Algenextrakten, Pilzextrakten, Pflanzenextrakten, Hefeextrakten, Bakterienextrakten; Enzymen; DHEA und seinen Derivaten und Metaboliten; antibakteriellen Wirkstoffen; Mattierungsmitteln; straffenden Wirkstoffen; und deren Gemischen ausgewählt ist.

30. Zusammensetzung nach dem vorhergehenden Anspruch, **dadurch gekennzeichnet, dass** der Wirkstoff unter Vitamin A, Vitamin C, Vitamin E, Vitamin B5, Vitamin B3, deren Estern und deren Gemischen ausgewählt ist.

31. Zusammensetzung nach Anspruch 29 oder 30, **dadurch gekennzeichnet, dass** der Wirkstoff unter Triclosan, Triclocarban, Salicylsäure und deren Gemischen ausgewählt ist.

32. Zusammensetzung nach einem der Ansprüche 29 bis 31, **dadurch gekennzeichnet, dass** der Wirkstoff ein Sonnenschutzfilter ist, das unter den organischen Filtern, physikalischen Filtern und deren Gemischen ausgewählt ist.

33. Zusammensetzung nach Anspruch 32, **dadurch gekennzeichnet, dass** das physikalische Filter unter den Oxiden von Titan, Oxiden von Zink und deren Gemischen ausgewählt ist.

34. Zusammensetzung nach Anspruch 28, **dadurch gekennzeichnet, dass** der schäumende grenzflächenaktive Stoff unter den nichtionischen, anionischen, amphoteren und zwitterionischen grenzflächenaktiven Stoffen und deren Gemischen ausgewählt ist.

35. Kosmetische und/oder dermatologische Zusammensetzung in Form einer Öl-in-Wasser-Emulsion, die eine in einer wässrigen Phase dispergierte Ölphase aufweist, **dadurch gekennzeichnet, dass** sie 0 bis etwa 1 Gew.-% emulgierenden grenzflächenaktiven aktiven Stoff, bezogen auf das Gesamtgewicht der Zusammensetzung, enthält, und **dadurch**, dass sie einer Zusammensetzung nach einem der vorhergehenden Ansprüche entspricht.

36. Zusammensetzung nach einem der vorhergehenden Ansprüche, **dadurch gekennzeichnet, dass** es sich um ein Produkt zur Pflege, zur Behandlung, zum Schutz, zur Reinigung, zum Abschminken und/oder zum Schminken von Keratinsubstanzen handelt.

37. Zusammensetzung nach dem vorhergehenden Anspruch, **dadurch gekennzeichnet, dass** die Keratinsubstanz die Haut ist.

38. Verwendung einer kosmetischen Zusammensetzung nach einem der Ansprüche 1 bis 35 als Produkt zur Pflege der Haut, der Haare, der Kopfhaut, der Wimpern, der Augenbrauen, der Nägel oder der Schleimhäute.

39. Verwendung einer kosmetischen Zusammensetzung nach einem der Ansprüche 1 bis 35 als Produkt zum Schminken.

40. Verwendung einer kosmetischen Zusammensetzung nach einem der Ansprüche 1 bis 35 als Produkt zum Sonnenschutz.

41. Verwendung einer kosmetischen Zusammensetzung nach einem der Ansprüche 1 bis 35 als Produkt für die Haarbehandlung, das ausgespült wird oder im Haar verbleibt.

42. Verwendung einer kosmetischen Zusammensetzung nach einem der Ansprüche 1 bis 35 als Produkt zur Reinigung und/oder zum Abschminken der Haut und/oder der Augen.

43. Verfahren zur kosmetischen Behandlung einer Keratinsubstanz, **dadurch gekennzeichnet, dass** auf die Keratinsubstanz eine kosmetische Zusammensetzung nach einem der Ansprüche 1 bis 35 aufgetragen wird.

44. Verfahren nach dem vorhergehenden Anspruch, **dadurch gekennzeichnet, dass** die Keratinsubstanz die Haut ist.

45. Verwendung mindestens eines wasserlöslichen oder in Wasser dispergierbaren Polymers mit Zweiblockstruktur A-B, wobei A ein ionischer wasserlöslicher Polymerblock ist und B einen hydrophoben Polymerblock bedeutet, wobei der Mengenanteil des Polymerblocks A mindestens 60 % des Gesamtgewichts des Zweiblockpolymers A-B beträgt, für die Gelbildung einer kosmetischen und/oder dermatologischen Zusammensetzung, die mindestens eine wässrige Phase und mindestens eine Ölphase enthält.

46. Verwendung nach dem vorhergehenden Anspruch, **dadurch gekennzeichnet, dass** der ionische Polymerblock A vollständig hydrophil und der Polymerblock B vollständig hydrophob ist.

47. Verwendung nach Anspruch 45 oder 46, **dadurch gekennzeichnet, dass** die Zusammensetzung ferner ein wasserlösliches oder in Wasser dispergierbares Zweiblockpolymer A'-B enthält, worin A' einen neutralen wasserlöslichen Polymerblock bedeutet und B ein hydrophober Polymerblock ist.
